# EUROPEAN PATENT APPLICATION

(11) **EP 4 600 330 A1**
(43) Date of publication of application: **13.08.2025**
(21) Application number: 23874947.7
(22) Date of filing: 05.10.2023
(51) Int. Cl.: C10G 2/00, C07C 1/04, C07C 11/06, C07C 15/02, C10L 3/12

(54) **METHOD FOR PRODUCING USEFUL HYDROCARBONS AND DEVICE FOR PRODUCING USEFUL HYDROCARBONS**

(30) Priority: 05.10.2022 JP 2022160891
(71) Applicant: FURUKAWA ELECTRIC CO., LTD., Tokyo 100-8322 (JP)
(72) Inventor: KAWAMATA, Yuki, Tokyo 100-8322 (JP); IWANO, Yuki, Tokyo 100-8322 (JP); TAKAHASHI, Hiroko, Tokyo 100-8322 (JP); BAMBA, Yuichiro, Tokyo 100-8322 (JP); HIRANO, Junya, Tokyo 100-8322 (JP); FUJIKAWA, Takashi, Tokyo 100-8322 (JP); LEE, Yu, Tokyo 100-8322 (JP); YAMANAKA, Nobumitsu, Tokyo 100-8322 (JP)
(74) Representative: Papula Oy
(86) International application number: PCT/JP2023/036448
(87) International publication number: WO 2024/075822

(57) **Abstract**

Provided is a useful hydrocarbon production method, the method comprising: a useful hydrocarbon generating step for generating, from a mixed gas comprising carbon monoxide and hydrogen, a first gas comprising useful hydrocarbon; a recycling step for separating, from the first gas, a dry reforming recycle gas comprising at least carbon dioxide and a low hydrocarbon-containing gas containing a low hydrocarbon; and a dry reforming step for generating, from the carbon dioxide and the low hydrocarbon-containing gas in the dry reforming recycle gas supplied from the recycling step, a second gas comprising carbon monoxide and hydrogen, and supplying the second gas to the useful hydrocarbon generating step.

## Description

### TECHNICAL FIELD

The present disclosure relates to a useful hydrocarbon production method and a useful hydrocarbon production device.

### BACKGROUND ART

Useful hydrocarbons such as various lower olefins and aromatic hydrocarbons which are basic chemical raw materials, various liquid fuels (gasoline or liquefied petroleum gas (LPG), aviation fuel, etc.) are hydrocarbons having a carbon number of 2 or higher, and are mainly produced from petroleum resources. However, due to the petroleum resources mainly being the raw materials in the production process of these hydrocarbons, there is concern over geopolitical risks. Furthermore, in a production process or consumption process of these hydrocarbons, due to discharging an abundance of carbon dioxide, it is considered to be a problem also from the viewpoint of global warming. Therefore, the development of a clean process establishing resources for which the regions of the reserves are not limited as raw materials, and having lower carbon dioxide emissions has been sought.

As an example, a useful hydrocarbon production method using hydrogen as a main raw material has been carried out. Hydrogen is a clean substance to the environment due to being generable from water. For example, Patent Document 1 discloses a method for producing liquefied petroleum gas from a synthesis gas. This production method includes: a first step of adding a hydrogen-containing gas to a synthesis gas; a second step of subjecting the gas after the first step to a reforming reaction to generate a lower paraffin-containing gas; a third step of separating a hydrogen-containing gas from the gas after the second step; a fourth step of separating a low boiling component gas containing carbon monoxide, methane and ethane from the gas after the third step; a fifth step of sequentially separating and extracting a target propane and butane from the gas after the fourth step; and a mole ratio adjusting step of adjusting a mole ratio of hydrogen to carbon monoxide to an optimum value for the reforming reaction by the first step adding the hydrogen-containing gas separated and recovered in the third step and/or fourth step.

However, byproducts such as carbon dioxide are generated in the above such production method, and thus the yield of useful hydrocarbons is low. In addition, since carbon dioxide is a root cause for global warming, the suppression of carbon dioxide emissions has been demanded.

Citation List

### Patent Document

Patent Document 1: Japanese Unexamined Patent Application, Publication No. 2011-213765

### DISCLOSURE OF THE INVENTION

### Problems to be Solved by the Invention

An object of the present disclosure is to provide a useful hydrocarbon production method and a useful hydrocarbon production device that can achieve an improvement in yield of useful hydrocarbons and suppression of carbon dioxide emissions, stably over a long period of time.

### Means for Solving the Problems

[1] A useful hydrocarbon production method includes: a useful hydrocarbon generating step of generating a first gas containing a useful hydrocarbon from a mixed gas containing carbon monoxide and hydrogen; a recycling step of separating a dry reforming recycle gas containing at least carbon dioxide and a lower hydrocarbon-containing gas containing a C1 to C4 lower hydrocarbon from the first gas; and a dry reforming step of generating a second gas containing carbon monoxide and hydrogen from the lower hydrocarbon-containing gas and carbon dioxide in the dry reforming recycle gas supplied from the recycling step, and supplying the second gas to the useful hydrocarbon generating step.
[2] The useful hydrocarbon production method as described in [1] further includes a raw material supplying step of supplying at least one of hydrogen and carbon monoxide that is obtained by reverse shift reaction to the useful hydrocarbon generating step.
[3] In the useful hydrocarbon production method as described in [1] or [2], the recycling step adjusts a ratio (M_{HC}/M_{CO2}) of a carbon mole number M_{HC} of the lower hydrocarbon-containing gas relative to a carbon mole number M_{CO2} of carbon dioxide in the dry reforming recycle gas to be supplied to the dry reforming step.
[4] In the useful hydrocarbon production method as described in any one of the above [1] to [3], a CO₂ concentration contained in the dry reforming recycle gas is 40% or more.
[5] The useful hydrocarbon production method as described in any one of the above [1] to [4] further includes a hydrogen recycling step of separating hydrogen from the first gas and supplying to the useful hydrocarbon generating step.
[6] In the useful hydrocarbon production method as described in [5], the hydrogen recycling step adjusts a supplied amount of hydrogen to be supplied to the useful hydrocarbon generating step, according to a molar amount of hydrogen supplied from the raw material supplying step and the dry reforming step.
[7] The useful hydrocarbon production method as described in any one of the above [1] to [6] further includes an adjusting step of adjusting a mole ratio (M_{H}/M_{CO}) of a mole number M_{H} of hydrogen relative to a mole number M_{CO} of carbon monoxide in the second gas obtained in the dry reforming step.
[8] The useful hydrocarbon production method as described in any one of the above [1] to [7] further includes a combusting step of separating a substance containing carbon atoms from the first gas and combusting the substance containing carbon atoms to generate carbon dioxide, in which the recycling step supplies the dry reforming recycle gas and carbon dioxide generated in the combusting step to the dry reforming step.
[9] In the useful hydrocarbon production method as described in any one of the above [1] to [8], the useful hydrocarbon is at least one type of hydrocarbon selected from the group consisting of olefins, aromatic hydrocarbons, gasoline, liquefied petroleum gas and liquid hydrocarbons.
[10] In the useful hydrocarbon production method as described in any one of the above [1] to [8], the useful hydrocarbon is liquefied petroleum gas.
[11] In the useful hydrocarbon production method as described in any one of the above [1] to [8], the useful hydrocarbon is liquefied petroleum gas, and hydrocarbons other than C3 to C4 hydrocarbons are included in the dry reforming recycle gas.
[12] In the useful hydrocarbon production method as described in any one of the above [1] to [8], the useful hydrocarbon is an olefin, and hydrocarbons other than a target olefin is included in the dry reforming recycle gas.
[13] In the useful hydrocarbon production method as described in the above [12], carbon monoxide is further included in the dry reforming recycle gas.
[14] In the useful hydrocarbon production method as described in any one of the above [1] to [8], the useful hydrocarbon is an aromatic hydrocarbon, and hydrocarbons other than a target aromatic hydrocarbon are included in the dry reforming recycle gas.
[15] In the useful hydrocarbon production method as described in the above [14], carbon monoxide is further included in the dry reforming recycle gas.
[16] In the useful hydrocarbon production method as described in any one of the above [1] to [8], the useful hydrocarbon is gasoline, and a hydrocarbon other than gasoline is included in the dry reforming recycle gas.
[17] In the useful hydrocarbon production method as described in the above [16], carbon monoxide is further included in the dry reforming recycle gas.
[18] In the useful hydrocarbon production method as described in any one of the above [1] to [8], the useful hydrocarbon is a liquid hydrocarbon, and C4 or lower hydrocarbons are included in the dry reforming recycle gas.
[19] In the useful hydrocarbon production method as described in the above [18], carbon monoxide is further included in the dry reforming recycle gas.
[20] A useful hydrocarbon production device includes: a useful hydrocarbon generating unit that generates a first gas containing a useful hydrocarbon from a mixed gas containing carbon monoxide and hydrogen; a recycling unit that separates a dry reforming recycle gas containing at least carbon dioxide and a lower hydrocarbon-containing gas containing a C1 to C4 lower hydrocarbon from the first gas; and a dry reforming unit that generates a second gas containing carbon monoxide and hydrogen from the lower hydrocarbon-containing gas and carbon dioxide in the dry reforming recycle gas supplied from the recycling unit, and supplies the second gas to the useful hydrocarbon generating unit.

### Effects of the Invention

According to the present disclosure, it is possible to provide a useful hydrocarbon production method and a useful hydrocarbon production device that can achieve an improvement in yield of useful hydrocarbons and suppression of carbon dioxide emissions, stably over a long period of time.

### BRIEF DESCRIPTION OF THE DRAWINGS

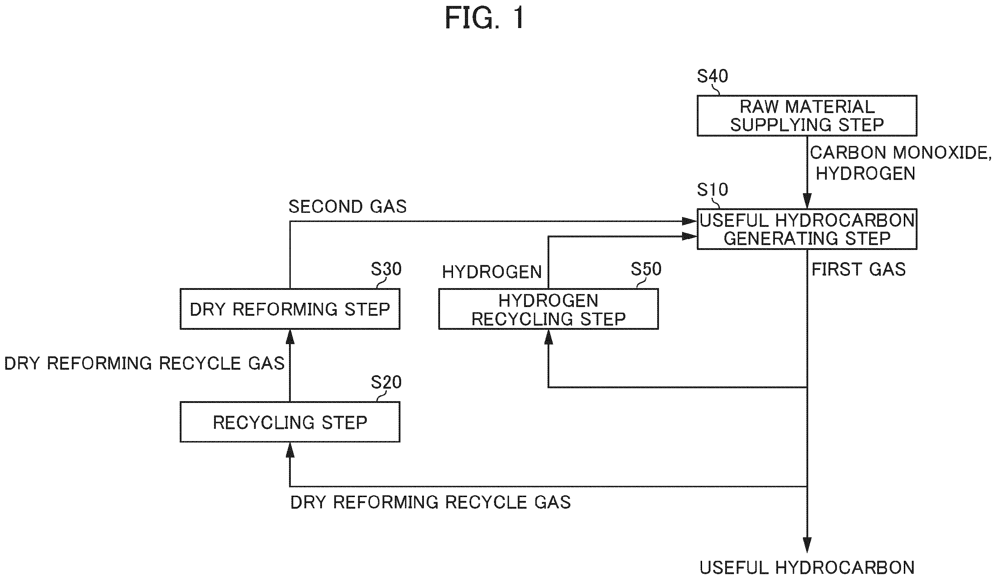

FIG. 1 is a block diagram showing an example of a useful carbon dioxide production method according to an embodiment.

### PREFERRED MODE FOR CARRYING OUT THE INVENTION

Hereinafter, an embodiment of the present invention will be described in detail while referencing the drawing.

As a result of thorough investigation, the present inventors have found that it is possible to achieve an improvement in yield of useful hydrocarbons and suppression of carbon dioxide emissions stably over a long period of time, by focusing on hydrogen which is a clean substance for the environment, using hydrogen as one of the main raw materials, and futher recycling carbon dioxide which is one of the principal byproducts within the system, thereby arriving at completion of the present disclosure based on this knowledge.

A useful hydrocarbon production method according to an embodiment includes: a useful hydrocarbon generating step of generating a first gas containing a useful hydrocarbon from a mixed gas containing carbon monoxide and hydrogen; a recycling step of separating, from the first gas, a dry reforming recycle gas containing at least carbon dioxide and a lower hydrocarbon-containing gas including C1 to C4 lower hydrocarbons; and a dry reforming step of generating a second gas containing carbon monoxide and hydrogen from the lower hydrocarbon-containing gas in the dry reforming recycle gas and carbon dioxide supplied from the recycling step, and supplying the second gas to the useful hydrocarbon generating step.

A useful hydrocarbon production device according to an embodiment includes: a useful hydrocarbon generating unit that generates a first gas containing a useful hydrocarbon from a mixed gas containing carbon monoxide and hydrogen; a recycling unit that separates, from the first gas, a dry reforming recycle gas containing at least carbon dioxide and a lower hydrocarbon-containing gas including C1 to C4 lower hydrocarbons; and a dry reforming unit that generates a second gas containing carbon monoxide and hydrogen from the lower hydrocarbon-containing gas in the dry reforming recycle gas and carbon dioxide supplied from the recycling unit, and supplies the second gas to the useful hydrocarbon generating unit.

### (Useful Hydrocarbon Production Method)

First, a useful hydrocarbon production method according to an embodiment will be described. FIG. 1 is a block diagram showing an example of a useful hydrocarbon production method according to the embodiment.

As shown in FIG. 1, the useful hydrocarbon production method includes a useful hydrocarbon generating step S10, a recycling step S20 and a dry reforming step S30.

The useful hydrocarbon generating step S10 generates a first gas containing a useful hydrocarbon from a mixed gas containing carbon monoxide and hydrogen. More specifically, in the useful hydrocarbon generating step S10, the first gas containing a useful hydrocarbon is generated from the carbon monoxide and hydrogen in the mixed gas, by way of the useful hydrocarbon synthesis reaction shown in Formula (1) below.

CO+2H₂-→ useful hydrocarbon ... Formula (1)

In this way, the useful hydrocarbon production method can produce the useful hydrocarbon using carbon monoxide and hydrogen as raw materials of the useful hydrocarbon synthesis reaction. For example, hydrogen can be generated from water, and carbon monoxide can be generated from carbon dioxide. In addition, the useful hydrocarbon may be separated from the first gas as necessary.

The useful hydrocarbon is contained in the first gas generated in the useful hydrocarbon generating step S10. Furthermore, in addition to the useful hydrocarbon, the first gas contains the lower hydrocarbon-containing gas, carbon dioxide, carbon monoxide and hydrogen, which are unreacted substances in the useful hydrocarbon generating step S10 and the dry reforming step S30 described later, and water, hydrocarbons other than the useful hydrocarbon, etc., which are byproducts of these steps. The lower hydrocarbon-containing gas is a gas under normal temperature and normal pressure, and contains C1-C4 hydrocarbons (hereinafter is also simply referred to as lower hydrocarbon).

The recycling step S20 performed after the useful hydrocarbon generating step S10 separates the dry reforming recycle gas containing at least the lower hydrocarbon-containing gas and carbon dioxide from the first gas generated in the useful hydrocarbon generating step S10, and supplies the dry reforming recycle gas to the dry reforming step S30.

The components of the dry reforming recycle gas are not particularly limited so long as containing the lower hydrocarbon-containing gas and carbon dioxide, and may be selected as appropriate. For example, the dry reforming recycle gas may be a gas obtained by separating the useful hydrocarbon from the first gas, or may be a gas obtained by extracting the lower hydrocarbon-containing gas and carbon dioxide from the first gas.

The dry reforming step S30 performed after the recycling step S20 generates a second gas containing carbon monoxide and hydrogen from the lower hydrocarbon-containing gas and carbon dioxide in the dry reforming recycle gas supplied from the recycling step S20, and supplies the second gas to the useful hydrocarbon generating step S10. The carbon monoxide and hydrogen in the second gas supplied to the useful hydrocarbon generating step S10 can be used as the raw materials of the useful hydrocarbon synthesis reaction carried out in the useful hydrocarbon generating step S10.

As described above, the recycling step S20 supplies the dry reforming recycle gas containing carbon dioxide and the lower hydrocarbon-containing gas which are impurities (off gas) in the first gas generated in the useful hydrocarbon generating step S10 to the dry reforming step S30, and can reuse the carbon monoxide and hydrogen generated in the dry reforming step S30 in the useful hydrocarbon generating step S10. In this way, the useful hydrocarbon production method of the embodiment can stably suppress carbon dioxide emissions over a long period of time, due to conducting recycle accompanying chemical conversion within the system of the carbon dioxide generated as a byproduct. In addition, in the recycling step S20, since the ratio of the carbon dioxide to lower hydrocarbon-containing gas which are byproducts separated from the first gas generated in the useful hydrocarbon generating step S10 is adjusted, and the carbon dioxide and lower hydrocarbon-containing gas are supplied as raw materials of the dry reforming step S30, degradation of the catalyst due to a phenomenon (so-called coking) in which coke precipitated by the polymerization of hydrocarbons is suppressed, and it is possible to stably improve the yield of the useful hydrocarbon over a long period of time.

Herein, among technologies for generating useful hydrocarbons, technology has been known that recycles, to a prior step, the off gas including the carbon dioxide contained in the gas containing the useful hydrocarbon, without performing chemical conversion to change the gas type. However, in the case of recycling the carbon dioxide as is, if there is a great amount of carbon dioxide, the generation efficiency of the useful hydrocarbon decreases, and as a result of this decrease in the generation efficiency, the discharge amount of carbon dioxide becomes great as a reaction system overall, and thus curbing the carbon dioxide has been a problem. On the other hand, the present disclosure found that it is possible to suppress a decrease in the generation efficiency of the useful hydrocarbon even if performing recycling, and thus suppress the discharge amount of carbon dioxide and suppress degradation of the catalyst, by supplying to the dry reforming step the off gas containing carbon dioxide and lower hydrocarbon-containing gas, which are off gases, and converting to carbon monoxide and hydrogen.

The lower hydrocarbon-containing gas in the dry reforming recycle gas supplied to the dry reforming step S30 may contain hydrocarbons other than the lower hydrocarbons, in addition to the lower hydrocarbons. The hydrocarbons other than the lower hydrocarbons contained in the lower hydrocarbon-containing gas are not particularly limited, and are pure substances of a C5 or higher hydrocarbon excluding the lower hydrocarbons and target useful hydrocarbon, or mixtures thereof, and are preferably C5 to C10 hydrocarbons excluding the lower hydrocarbons and the target useful hydrocarbon. At this time, the ratio of the mole number of the lower hydrocarbons relative to the mole number of C5 or higher hydrocarbons excluding the lower hydrocarbons and target useful hydrocarbon in the lower hydrocarbon-containing gas (lower hydrocarbons/C5 or higher hydrocarbons excluding lower hydrocarbons and useful hydrocarbon) is preferably 1.0 or higher. If the dry reforming recycle gas containing the lower hydrocarbon-containing gas of such a composition is supplied to the dry reforming step S30, the coking of the dry reforming catalyst is effectively suppressed, and the yield of the useful hydrocarbon generated in the useful hydrocarbon generating step S10 improves. In particular, since the content is great and the generation of the coking is also low, ethane is preferable even among lower hydrocarbons.

In addition, the target useful hydrocarbon may be contained in a trace amount in the hydrocarbons other than the lower hydrocarbons contained in the dry reforming recycle gas, and the amount thereof is acceptable if smaller than the generated amount of the target useful hydrocarbon in the first gas.

The dry reforming step S30 synthesizes carbon monoxide and hydrogen from the ethane and carbon dioxide contained in the dry reforming recycle gas by dry reforming as in the following Formula (2), for example. In addition, since the hydrocarbons serving as raw materials of dry reforming are not limited to ethane, when generalizing Formula (2) to saturated hydrocarbons, for example, it becomes the following Formula (3). At this time, carbon contained in the hydrocarbons and carbon dioxide as raw materials react in equal amounts.

C₂H₆+2CO₂→4CO+3H₂ ... Formula (2)

CₙH₂ₙ₊₂+nCO₂→2nCO+ (n+1)H₂ (n≧1) ... Formula (3)

In the dry reforming step S30, a catalyst for synthesizing carbon monoxide and hydrogen, by dry reforming, from the lower hydrocarbon-containing gas and carbon dioxide contained in the dry reforming recycle gas (hereinafter also simply referred to as dry reforming catalyst) is used. By heating the dry reforming catalyst while supplying the dry reforming recycle gas to the dry reforming catalyst in the dry reforming step S30, the lower hydrocarbon-containing gas and carbon dioxide in the supplied dry reforming recycle gas reacts on the dry reforming catalyst to generate the second gas containing carbon monoxide and hydrogen.

The dry reforming catalyst is not particularly limited; however, from the viewpoint of exhibiting catalytic activity over a long period of time to generate carbon monoxide and hydrogen, for example, the dry reforming catalyst is preferably a catalyst structure including a carrier of a porous structure configured by a zeolite-type compound, and at least one catalytic substance existing within the carrier, in which the carrier has passages communicating with each other, the ratio (L/d ratio) of the long-side dimension L of the carrier relative to the thickness dimension d of the carrier is 5.0 or more, and the catalytic substance exists at least in the passages of the carrier.

Regarding such a catalyst structure, the L/d ratio of the carrier is 5.0 or more, preferably 5.0 or more and 35.0 or less, and more preferably 7.0 or more and 25.0 or less. When the L/d ratio of the carrier is 5.0 or more, it is possible to improve the catalytic activity. In addition, when the L/d ratio is 35.0 or less, it is possible to improve the manufacturing yield of the catalyst structure.

In addition, regarding such a catalyst structure, the average particle size of the catalytic substance is preferably 0.08 nm or more and 50.00 nm or less. When the average particle size of the catalytic substance is within the above-mentioned range, the catalytic activity sufficiently increases, and it is possible to improve the catalytic activity as the average particle size decreases. In addition, from the viewpoint of a balance of high catalytic activity and the coking resistance (catalyst deterioration resistance), the average particle size of the catalytic substance is preferably 9.00 nm or less, and more preferably 4.50 nm or less.

In addition, so long as being a catalyst which is well known as a dry reforming catalyst, it can be utilized as the dry reforming catalyst. For example, as described in Yuche Gao et al., A review of recent developments in hydrogen production via biogas dry reforming, Energy Conversion and Management, 171 (2018) 133-155, it is possible to include at least one type selected from Ir, Ru, Rh, Pt, Pd, Ni, Co, and Fe, which are metals having activity in dry reforming, and as the carrier, to use a carrier consisting of at least one type selected from MgO, Al₂O₃,SiO₂, CeO₂, CaO, ZrO₂, TiO₂, La₂O₃, ZnO, silicalite-1, MCM-41 and SBA-15. However, the dry reforming catalyst is not particularly limited to these.

The heating temperature of the dry reforming catalyst is set as appropriate according to the type of dry reforming catalyst, supplied amount of dry reforming recycle gas, content ratio of lower hydrocarbon-containing gas to carbon dioxide contained in the dry reforming recycle gas, etc. For example, for the heating temperature of the dry reforming catalyst, the lower limit value is preferably 400°C or higher, and more preferably 600°C or higher, and the upper limit value is preferably 1000°C or lower, and more preferably 900°C or lower.

A synthesis gas containing at least carbon monoxide and hydrogen is contained in the second gas generated in the dry reforming step S30. In addition to carbon monoxide and hydrogen (synthesis gas), hydrocarbons and carbon dioxide that are unreacted substances, water which is a byproduct, etc., are contained in the second gas.

The content ratio of carbon monoxide and hydrogen contained in the second gas can be appropriately adjusted according to dry reforming conditions such as the heating temperature of the dry reforming catalyst, supplied amount of the dry reforming recycle gas described later, and content ratio of lower hydrocarbon-containing gas and carbon dioxide contained in the dry reforming recycle gas.

In addition, the recycling step S20 preferably adjusts the ratio (M_{HC}/M_{CO2}) of the carbon mole number M_{HC} of the lower hydrocarbon-containing gas relative to the carbon mole number M_{CO2} of carbon dioxide in the dry reforming recycle gas supplied to the dry reforming step S30. For example, it is possible to adjust the ratio (M_{HC}/M_{CO2}) by the adjustment of at least one of the amount of lower hydrocarbon-containing gas and the amount of carbon dioxide contained in the dry reforming recycle gas.

As shown in Formulas (2) and (3) above, in the dry reforming step S30, carbon monoxide and hydrogen are generated from the lower hydrocarbon-containing gas and carbon dioxide (1:1 carbon mole ratio) in the same ratio (carbon basis). On the other hand, in the dry reforming step S30, when the ratio (M_{HC}/M_{CO2}) of the carbon mole number M_{HC} of the lower hydrocarbon-containing gas relative to the carbon mole number M_{CO2} of carbon dioxide becomes large, the coking of the dry reforming catalyst tends to occur, and as a result, the activity of the dry reforming catalyst declines, and the generated amount of the useful hydrocarbon decreases. Furthermore, since the carbon dioxide is deficient, the efficiency of dry reforming declines.

For this reason, the recycling step S20, when the ratio (M_{HC}/M_{CO2}) in the dry reforming step S30 is large, can suppress the coking occurrence in the dry reforming step S30 by carrying out a decrease in the low hydrocarbon-containing gas amount in the dry reforming recycle gas or increase in carbon dioxide amount in the dry reforming recycle gas to decrease the ratio (M_{HC}/M_{CO2}) in the dry reforming recycle gas, and thus further can suppress the carbon dioxide deficiency, and improve the efficiency of dry reforming. If adjusting the ratio (M_{HC}/M_{CO2}) in the dry reforming recycle gas preferably to 1.30 or less, and more preferably to 1.10 or less, the above effect can be further improved.

In addition, when adjusting the ratio (M_{HC}/M_{CO2}) in the dr reforming recycle gas to 0.25 or more, and more preferably to 0.40 or more, it is possible to suppress a lower hydrocarbon-containing gas deficiency, and thus improve the efficiency of the dry reforming. Furthermore, the discharge amount of carbon dioxide can be further suppressed. In the case of the ratio (M_{HC}/M_{CO2}) in the dry reforming step S30 being small, the recycling step S20 increases the ratio (M_{HC}/M_{CO2}) by making an increase in the lower hydrocarbon-containing gas amount in the dry reforming recycle gas or a decrease in the carbon dioxide amount in the dry reforming recycle gas.

In addition, the CO₂ concentration of the dry reforming recycle gas supplied to the dry reforming step S30 by the recycling step S20 is preferably 40% or more, more preferably 50% or more, and even more preferably 60% or more. When the CO₂ concentration of the dry reforming recycle gas is 40% or more, it is possible to sufficiently suppress the coking occurrence and the carbon dioxide deficiency in the dry reforming step S30.

Furthermore, by eliminating the carbon dioxide deficiency in the dry reforming step S30, the efficiency of dry reforming is improved, and as a result, since the existence ratio of carbon monoxide and hydrogen in the second gas increases, the generation efficiency of the useful hydrocarbon improves.

In addition, the useful hydrocarbon production method of the embodiment preferably further includes a raw material supplying step S40 of supplying at least one of hydrogen and carbon monoxide that is obtained by the reverse shift reaction to the useful hydrocarbon generating step S10. The hydrogen and carbon monoxide supplied from the raw material supplying step S40 to the useful hydrocarbon generating step S10 can be used as raw materials of the useful hydrocarbon synthesis reaction carried out in the useful hydrocarbon generating step S10.

Carbon monoxide is generated from carbon dioxide and hydrogen by way of the reverse shift reaction (CO₂+H₂→CO+H₂O). In addition, hydrogen is generated from the electrolysis of water, etc., for example. In this way, the raw material supplying step S40 can stably supply hydrogen and carbon monoxide to the useful hydrocarbon generating step S10.

In addition, the useful hydrocarbon production method of the embodiment preferably further includes a hydrogen recycling step S50 of separating hydrogen from the first gas generated in the useful hydrocarbon generating step S10 and supplying to the useful hydrocarbon generating step S10.

As shown in the above Formula (1), in the useful hydrocarbon generating step S10, twice the amount of hydrogen relative to carbon monoxide is required. On the other hand, a great amount of carbon monoxide relative to hydrogen is contained in the second gas generated in the dry reforming step S30. In this way, since twice as much hydrogen than carbon monoxide is required in the useful hydrocarbon generating step S10, and more carbon monoxide than hydrogen is generated in the dry reforming step S30, if carrying out the useful hydrocarbon generating step S10 for a long period of time, it may lead to a hydrogen deficiency. As a result thereof, since carbon monoxide becomes excessive, the generated amount of the useful hydrocarbon obtained in the useful hydrocarbon generating step S10 declines.

Therefore, in the hydrogen recycling step S50, by supplying to the useful hydrocarbon generating step S10 hydrogen, which is an impurity (off gas) in the first gas generated in the useful hydrocarbon generating step S10, it is possible to compensate for the insufficient amount of hydrogen, which is the raw material for the useful hydrocarbon synthesis reaction conducted in the useful hydrocarbon generating step S10. In this way, by way of the hydrogen recycling step S50, since it is possible to suppress the hydrogen deficiency due to the useful hydrocarbon generating step S10 over a long period of time, the useful hydrocarbon generating step S10 can be stably performed over a long period of time. In addition, since the hydrogen discharged from the useful hydrocarbon generating step S10 is reused as a raw material of the useful hydrocarbon generating step S10, the environmental burden can be reduced.

The above-mentioned hydrogen deficiency stems from the technology of the present disclosure in converting the lower hydrocarbon-containing gas and carbon dioxide to carbon monoxide and hydrogen in the dry reforming step, and then supplying to the useful hydrocarbon generating step, as is evident in that carbon monoxide is more abundant comparing the mole ratio of carbon monoxide to hydrogen in Formula (2) and Formula (3). By including the hydrogen recycling step S50, the present disclosure can eliminate the hydrogen deficiency in the dry reforming step S30, and more stably generate the useful hydrocarbon.

In addition, the hydrogen recycling step S50 preferably adjusts the supplied amount of hydrogen to be supplied in the useful hydrocarbon generating step S10 according to the molar amount of hydrogen supplied from the raw material supplying step S40 and the dry reforming step S30, and more preferably adjusts the supplied amount of hydrogen to be supplied to the useful hydrocarbon generating step S10, according to the carbon number of the useful hydrocarbon generated in the useful hydrocarbon generating step S10 and the mole number M_{H} of hydrogen in the useful hydrocarbon generating step S10.

In the case of producing liquefied petroleum gas as the useful hydrocarbon, for example, if the mole number M_{H} of hydrogen in the useful hydrocarbon generating step S10 is large, the useful hydrocarbon synthesis reaction is favorably carried out. On the other hand, if the mole number M_{H} of hydrogen in the useful hydrocarbon generating step S10 is small, there is a possibility of the hydrogen deficiency arising in the useful hydrocarbon synthesis reaction. For this reason, when the mole number M_{H} of hydrogen in the useful hydrocarbon generating step S10 is small, if the hydrogen recycling step S50 increases the supplied amount of hydrogen to be supplied to the useful hydrocarbon generating step S10, it is possible to suppress the hydrogen deficiency in the useful hydrocarbon generating step S10.

In this way, it is possible to more efficiently produce the target useful hydrocarbon by adjusting, in the hydrogen recycling step S50, the supplied amount of hydrogen to be supplied to the useful hydrocarbon generating step S10 according to the mole number M_{H} of hydrogen in the useful hydrocarbon generating step S10, such as increasing the supplied amount of hydrogen to be supplied to the useful hydrocarbon generating step S10 when the mole number M_{H} of hydrogen in the useful hydrocarbon generating step S10 is small.

In addition, the hydrogen recycling step S50 preferably adjusts the supplied amount of hydrogen to be supplied to the useful hydrocarbon generating step S10 according to the mole ratio (M_{H}/M_{CO}) of the mole number M_{H} of hydrogen relative to the mole number M_{CO} of carbon monoxide in the useful hydrocarbon generating step S10.

Regarding the mole ratio (M_{H}/M_{CO}) in the useful hydrocarbon generating step S10, if the proportion of hydrogen relative to carbon monoxide is small, there is a possibility of the hydrogen deficiency arising. For this reason, when the mole ratio (M_{H}/M_{CO}) in the useful hydrocarbon generating step S10 is small, if the hydrogen recycling step S50 increases the supplied amount of hydrogen to be supplied to the useful hydrocarbon generating step S10, the hydrogen recycling step S50 can suppress the hydrogen deficiency in the useful hydrocarbon generating step S10. In this way, by the hydrogen recycling step S50 adjusting the supplied amount of hydrogen to be supplied to the useful hydrocarbon generating step S10 according to the mole ratio (M_{H}/M_{CO}) in the useful hydrocarbon generating step S10, it is possible to more reliably suppress the hydrogen deficiency in the useful hydrocarbon generating step S10.

In addition, the useful hydrocarbon production method of the embodiment may further include an adjusting step (not shown) of adjusting the mole ratio (M_{H}/M_{CO}) of the mole number M_{H} of hydrogen relative to the mole number M_{CO} of carbon monoxide in the second gas obtained in the dry reforming step S30.

The adjusting step adjusts the mole ratio (M_{H}/M_{CO}) in the second gas obtained in the dry reforming step S30 to 1 or more and 3 or less by supplying steam to the dry reforming step S30. In the adjusting step, the mole ratio (M_{H}/M_{CO}) in the second gas is adjusted according to the conditions of the useful hydrocarbon synthesis reaction conducted in the useful hydrocarbon generating step S10.

If the second gas having the mole ratio (M_{H}/M_{CO}) adjusted by the adjusting step is supplied to the useful hydrocarbon generating step S10, since it is possible to suppress the hydrogen deficiency due to the useful hydrocarbon generating step S10 over a long period of time, the useful hydrocarbon generating step S10 can be stably performed over a long period of time. For this reason, the target useful hydrocarbon can be efficiently produced over a long period of time.

In addition, the useful hydrocarbon production method of the embodiment may further include a combusting step (not shown) of separating a substance containing carbon atoms from the first gas, and combusting the substance containing carbon atoms to generate carbon dioxide. In this case, the recycling step S20 supplies the dry reforming recycle gas and the carbon dioxide generated in the combusting step to the dry reforming step S30.

For example, in the case of producing liquefied petroleum gas as the useful hydrocarbon, in addition to the liquefied petroleum gas, the first gas discharged from the useful hydrocarbon generating step S10 contains, as impurities, substances containing carbon atoms such as ethane. The combusting step separates substances containing carbon atoms in the first gas discharged from the useful hydrocarbon generating step S10 from the first gas, and combusts the separated substances containing carbon atoms to generate carbon dioxide. It should be noted that carbon dioxide may or may not being included in the substances containing carbon atoms combusting in the combusting step.

The recycling step S20 supplies the carbon dioxide generated in the combusting step to the dry reforming step S30, in addition to the dry reforming recycle gas. The amount of carbon dioxide supplied to the dry reforming step S30 can be increased by the combusting step. For this reason, since the coking occurrence and the carbon dioxide deficiency in the dry reforming step S30 can be further suppressed, the target useful hydrocarbon can be efficiently produced over a long period of time. In addition, it is possible to recover the heat generated in the combusting step, and utilize the heat in the useful hydrocarbon generating step S10 and/or the dry reforming step S30.

In addition, it is preferable to adjust the combustion rate of the combusting step, according to the proportion of hydrocarbons to carbon dioxide supplied to the dry reforming step S30. The combustion rate of the combusting step is the proportion of combusting hydrocarbons excluding the target useful hydrocarbon component in the first gas to convert to carbon dioxide as shown in Formula (4) below, and can be calculated from the following Formula. For example, in the case of the combustion rate of the combusting step being 100%, the combusting step generates carbon dioxide by combusting all hydrocarbons excluding the target useful hydrocarbon component in the first gas.

2CₙH₂ₙ₊₂+(3n+1)O₂→(2n+2)H₂O+2nCO₂ ... Formula (4)

Combustion rate (%) = 100 - (total hydrocarbon amount (C-mol) excluding target useful hydrocarbon component combusted in combusting step x 100 / total hydrocarbon amount (C-mol) excluding target useful hydrocarbon component in first gas)

In this way, it is possible to adjust the amount of carbon dioxide generated in the combusting step, by varying the combustion rate of the combusting step. For this reason, since it is possible to adjust the combustion rate of the combusting step to adjust the generated amount of carbon dioxide according to the proportion of hydrocarbons to carbon dioxide supplied to the dry reforming step S30, and possible to adjust the amount of carbon dioxide supplied to the dry reforming step S30 by the recycling step S20, the generated amount of the target useful hydrocarbon can be increased.

For example, in the case of producing liquefied petroleum gas in the useful hydrocarbon generating step S10, if the combustion rate of the combusting step is 3% or more, the carbon dioxide deficiency in the dry reforming step S30 can be sufficiently suppressed. In addition, if the combustion rate of the combusting step is 25% or less, the proportion of hydrocarbons to carbon dioxide supplied to the dry reforming step S30 will be favorable. On the other hand, if the combustion rate of the combusting step exceeds 25%, since carbon dioxide becomes excessive relative to hydrocarbons, the unreacted carbon dioxide amount becomes great, and as a result, the generated amount of the useful hydrocarbon decreases. For this reason, if the combustion rate of the combusting step is 3% or more and 25% or less, the useful hydrocarbon can be efficiently produced over a long period of time.

In addition, in the useful hydrocarbon production method of the embodiment, trace amounts of sulfur and halogens may be mixed as impurities in each gas flowing in the system. Since these become poisonous substances for the useful hydrocarbon generation catalyst used in the useful hydrocarbon generating step S10 and the dry reforming catalyst used in the dry reforming step S30, there is a possibility of hindering the catalytic performance of the above catalysts, and may become the cause of damage to equipment. Therefore, the useful hydrocarbon production method of the embodiment may include a desulfurization step, dehalogenation step or the like as appropriate.

In addition, the useful hydrocarbon production method of the embodiment may include a dehydrating step (not shown) of dehydrating the second gas generated in the dry reforming step S30. The dehydrating step is performed after the dry reforming step S30 and before the useful hydrocarbon generating step S10. Water may be contained in the second gas supplied to the useful hydrocarbon generating step S10. By dehydrating the second gas in the dehydrating step, it is possible to improve the efficiency of the useful hydrocarbon synthesis reaction performed in the useful hydrocarbon generating step S10, and possible to further suppress a decline in catalytic performance of the useful hydrocarbon generation catalyst due to moisture.

In addition, the useful hydrocarbon production method of the embodiment preferably includes a separating step after the raw material supplying step S40 and before the useful hydrocarbon generating step S10. In the case of the raw material supplying step S40 supplying the carbon monoxide obtained by the reverse shift reaction to the useful hydrocarbon generating step S10, hydrogen and/or carbon dioxide, water, etc. are contained as unreacted substances and byproducts in the gas generated from the raw material supplying step S40. For this reason, a part or all of the hydrogen and carbon dioxide may be separated from carbon monoxide and recycled as appropriate to the raw material supplying step S40. In addition, it is preferable to separate water from the carbon monoxide, due to being possible to improve the efficiency of the useful hydrocarbon synthesis reaction, and being further possible to suppress a decline in the catalytic performance of the useful hydrocarbon generation catalyst due to moisture.

In addition, the useful hydrocarbon production method of the embodiment may include a compressing step (not shown) of compressing the second gas generated in the dry reforming step S30, as well as a gas containing at least one of hydrogen and carbon monoxide supplied from the raw material supplying step S40. The compressing step is performed after the dry reforming step S30 and before the useful hydrocarbon generating step S10 in the compression of the second gas, and is performed after the raw material supplying step S40 and before the useful hydrocarbon generating step S10 in the compression of the above-mentioned gas. By compressing the second gas, etc. in the compressing step, since the compressed second gas, etc. is supplied to the useful hydrocarbon generating step S10, the efficiency of the useful hydrocarbon synthesis reaction performed in the useful hydrocarbon generating step S10 can be improved.

In addition, the useful hydrocarbon produced by the useful hydrocarbon production method is preferably at least one type of hydrocarbon selected from the group consisting of olefins, aromatic hydrocarbons, gasoline, liquefied petroleum gas and liquid hydrocarbons, and is more preferably liquefied petroleum gas.

As the olefins, ethylene and propylene are preferable. As the aromatic hydrocarbons, benzene, toluene and xylene are preferable. As gasoline, C5 to C12 hydrocarbons are preferable. As the liquefied petroleum gas, C3 to C4 hydrocarbons are preferable. As the liquid hydrocarbons, naphtha, kerosene, jet fuel, light oil and heavy oil, which are C5 or higher hydrocarbons, are preferable.

The useful hydrocarbon production method can synthesize a hydrocarbon mixture with various target useful hydrocarbons as the main component, from carbon monoxide and hydrogen, by various useful hydrocarbon synthesis reactions, with the appropriate catalyst (useful hydrocarbon generation catalyst) and reaction conditions in the useful hydrocarbon generating step S10, according to the type of useful hydrocarbon to be produced.

For example, in the case of producing olefins as the useful hydrocarbon, olefin synthesis reaction is performed using the useful hydrocarbon generation catalyst (olefin generation catalyst) for synthesizing the olefins from carbon monoxide and hydrogen contained in the mixed gas in the useful hydrocarbon generating step S10. In the useful hydrocarbon generating step S10, by heating the olefin generation catalyst while supplying the mixed gas to the olefin generation catalyst, the carbon monoxide and hydrogen in the supplied mixed gas react on the olefin generation catalyst to generate the first gas containing olefins.

From the viewpoint of increasing the generated amount of olefins, the olefin generation catalyst is preferably a catalyst including a methanol synthesis catalytic substance and a ZSM-5-type zeolite catalytic substance.

For such a catalyst, the ZSM-5-type zeolite catalytic substance preferable contains P (phosphorus). If the ZSM-5-type zeolite catalytic substance includes P, since the acid strength of the ZSM-5-type zeolite catalytic substance can be properly controlled, it is possible to curb the growth of excessive hydrocarbon chains, and increase the generated amount of olefins such as ethylene and propylene.

In addition, for such a catalyst, the ratio (mole number of SiO₂/mole number of Al₂O₃) of the mole number of SiO₂ relative to the mole number of Al₂O₃ contained in the ZSM-5-type zeolite catalytic substance is preferably 20 or more and 60 or less. By replacing a part of the silicon atoms in the silicates constituting the zeolite skeleton of the ZSM-5-type zeolite catalytic substance with aluminum atoms, since the aluminum atoms serve as acid sites, the zeolite catalytic substance exhibits a function as a solid acid. If the above ratio is 60 or less, since the acid sites of the ZSM-5-type zeolite catalytic substance increase, it is possible to increase the generated amount of olefins. In addition, if the above ratio is 20 or more, it is possible to suppress catalyst degradation such as coking caused by the acid sites becoming too abundant.

The heating temperature of the olefin generation catalyst is appropriately set according to the type of olefin generation catalyst, supplied amount of the mixed gas, etc. For example, the heating temperature of the olefin generation catalyst is 240°C or higher and 350°C or lower.

For the pressure of the olefin synthesis reaction, the lower limit value is preferably 2.0 MPa or more, more preferably 3.0 MPa or more, and even more preferably 3.5 MPa or more, and the upper limit value is preferably 6.0 MPa or less, more preferably 5.5 MPa or less, and even more preferably 5.0 MPa or less.

At this time, at least olefins which are useful hydrocarbon are contained in the first gas generated in the useful hydrocarbon generating step S10. The first gas contains, in addition to olefins, the hydrocarbons, carbon dioxide, carbon monoxide and hydrogen that are unreacted substances of the useful hydrocarbon generating step S10 and the dry reforming step S30, and methanol, dimethyl ether, C10 or lower hydrocarbons, etc., which are byproducts of these steps.

The content ratio of olefins contained in the first gas can be appropriately adjusted according to the conditions of the olefin synthesis reaction such as the heating temperature of the olefin generation catalyst and the supplied amount of the mixed gas.

In the case of the useful hydrocarbon being olefins, hydrocarbons other than the target olefins are preferably contained in the dry reforming recycle gas, and hydrocarbons other than the target olefins and carbon monoxide are more preferably contained therein. If the dry reforming recycle gas contains the above-mentioned substances, the yield of olefins drastically improves.

For example, in the case of producing aromatic hydrocarbons as the useful hydrocarbon, the aromatic hydrocarbon synthesis reaction is performed using the useful hydrocarbon generation catalyst (aromatic hydrocarbon generation catalyst) for synthesizing the aromatic hydrocarbon from the carbon monoxide and hydrogen contained in the mixed gas in the useful hydrocarbon generating step S10. In the useful hydrocarbon generating step S10, by heating the aromatic hydrocarbon generation catalyst while supplying the mixed gas to the aromatic hydrocarbon generation catalyst, the carbon monoxide and hydrogen in the supplied mixed gas react on the aromatic hydrocarbon generation catalyst to generate a first gas containing the aromatic hydrocarbon.

From the viewpoint of increasing the generated amount of aromatic hydrocarbons, the aromatic hydrocarbon generation catalyst is preferably a catalyst including a methanol synthesis catalytic substance and a ZSM-5-type zeolite catalytic substance.

For such a catalyst, it is preferable to support, as the active metal oxide, at least one among MnO, MnCr₂O₄, MnAl₂O₄, MnZrO₄, ZnO, ZnCr₂O₄ and ZnAl₂O₄ on the ZSM-5-type zeolite catalytic substance, and it is more preferable to support at least one among MnO, MnCr₂O₄, MnAl₂O₄, and MnZrO₄ thereon. If the ZSM-5-type zeolite catalytic substance supports the above-mentioned metal oxide, it is possible to increase the generated amount of aromatic hydrocarbons.

In addition, for such a catalyst, the ratio (mole number of SiO₂/ mole number of Al₂O₃) of the mole number of SiO₂ relative to the mole number of Al₂O₃ contained in the ZSM-5-type zeolite catalytic substance is preferably 20 or more and 60 or less. By replacing a part of the silicon atoms in the silicates constituting the zeolite skeleton of the ZSM-5-type zeolite catalytic substance with aluminum atoms, since the aluminum atoms serve as acid sites, the zeolite catalytic substance exhibits a function as a solid acid. If the above ratio is 60 or less, since the acid sites of the ZSM-5-type zeolite catalytic substance increase, it is possible to increase the generated amount of aromatic hydrocarbons. In addition, if the above ratio is 20 or more, it is possible to suppress catalyst degradation such as coking caused by the acid sites becoming too abundant.

The heating temperature of the aromatic hydrocarbon generation catalyst is appropriately set according to the type of aromatic hydrocarbon generation catalyst, supplied amount of the mixed gas, etc. For example, the heating temperature of the aromatic hydrocarbon generation catalyst is 300°C or higher and 600°C or lower.

The pressure of the aromatic hydrocarbon synthesis reaction is preferably 0.1 MPa or more and 6.0 MPa or less.

At this time, at least the aromatic hydrocarbon which is the useful hydrocarbon is contained in the first gas generated in the useful hydrocarbon generating step S10. In addition to the aromatic hydrocarbon, the first gas contains hydrocarbons, carbon dioxide, carbon monoxide and hydrogen that are unreacted substances of the useful hydrocarbon generating step S10 and the dry reforming step S30, and methanol, dimethyl ether, hydrocarbons other than the aromatic hydrocarbon that is the useful hydrocarbon, etc. which are byproducts of these steps.

The content ratio of aromatic hydrocarbons contained in the first gas can be appropriately adjusted according to the conditions of the aromatic hydrocarbon synthesis reaction such as the heating temperature of the aromatic hydrocarbon generation catalyst and the supplied amount of the mixed gas.

In the case of the useful hydrocarbon being an aromatic hydrocarbon, the hydrocarbons other than the target aromatic hydrocarbon are preferably contained in the dry reforming recycle gas, and hydrocarbons other than the target aromatic hydrocarbon and carbon monoxide are more preferably contained therein. If the dry reforming recycle gas contains the above-mentioned substances, the yield of aromatic hydrocarbons drastically improves.

For example, in the case of producing gasoline as the useful hydrocarbon, the gasoline synthesis reaction is performed using the useful hydrocarbon generation catalyst (gasoline generation catalyst) for synthesizing gasoline from the carbon monoxide and hydrogen contained in the mixed gas in the useful hydrocarbon generating step S10. In the useful hydrocarbon generating step S10, by heating the gasoline generation catalyst while supplying the mixed gas to the gasoline generation catalyst, the carbon monoxide and hydrogen in the supplied mixed gas react on the gasoline generation catalyst to generate the first gas containing gasoline.

From the viewpoint of increasing the generated amount of gasoline, the gasoline generation catalyst is preferably a catalyst including a methanol synthesis catalytic substance and a zeolite catalytic substance.

In addition, for such a catalyst, the ratio (mole number of SiO₂/mole number of Al₂O₃) of the mole number of SiO₂ relative to the mole number of Al₂O₃ contained in the zeolite catalytic substance is preferably at least 12. In addition, the zeolite catalytic substance has a pore diameter formed preferably by up to 12-membered rings, and more preferably by up to 10-membered rings. By replacing a part of the silicon atoms in the silicates constituting the zeolite skeleton of the zeolite catalytic substance with aluminum atoms, since the aluminum atoms serve as acid sites, the zeolite catalytic substance exhibits a function as a solid acid. If the above such zeolite catalytic substance, it is possible to increase the generated amount of gasoline. Such a zeolite catalytic substance is, for example, ZSM-5, ZSM-11, ZSM-12, ZSM-23, ZSM-35 and ZSM-38.

The heating temperature of the gasoline generation catalyst is appropriately set according to the type of gasoline generation catalyst, the supplied amount of the mixed gas, etc. For example, the heating temperature of the gasoline generation catalyst is 250°C or higher and 500°C or lower, and preferably 300°C or higher and 450°C or lower.

The pressure of the gasoline synthesis reaction is preferably 25 bar or more and 150 bar or less.

At this time, at least gasoline which is the useful hydrocarbon is contained in the first gas generated in the useful hydrocarbon generating step S10. In addition to the gasoline, the first gas contains hydrocarbons, carbon dioxide, carbon monoxide and hydrogen that are unreacted substances of the useful hydrocarbon generating step S10 and the dry reforming step S30, and methanol, dimethyl ether, hydrocarbons other than gasoline, etc. which are byproducts of these steps.

The content ratio of gasoline contained in the first gas can be appropriately adjusted according to the conditions of the gasoline synthesis reaction such as the heating temperature of the gasoline generation catalyst and the supplied amount of the mixed gas.

In the case of the useful hydrocarbon being gasoline, hydrocarbons other than the target gasoline are preferably contained in the dry reforming recycle gas, and hydrocarbons other than the target gasoline and carbon monoxide are more preferably contained therein. If the dry reforming recycle gas contains the above-mentioned substances, the yield of gasoline drastically improves.

For example, in the case of producing liquefied petroleum gas as the useful hydrocarbon, the liquefied petroleum gas synthesis reaction is performed using the useful hydrocarbon generation catalyst (LPG generation catalyst) for synthesizing liquefied petroleum gas from the carbon monoxide and hydrogen contained in the mixed gas in the useful hydrocarbon generating step S10. In the useful hydrocarbon generating step S10, by heating the LPG generation catalyst while supplying the mixed gas to the LGP generation catalyst, the carbon monoxide and hydrogen in the supplied mixed gas react on the LPG generation catalyst to generate the first gas containing liquefied petroleum gas.

From the viewpoint of increasing the generated amount of liquefied petroleum gas and increasing the content ratio of propane contained in the liquefied petroleum gas, the LPG generation catalyst is preferably a catalyst including a methanol synthesis catalytic substance and a ZSM-5-type zeolite catalytic substance.

For such a catalyst, Pt (platinum) or Pt and Pd (palladium) are preferably supported on the ZSM-5-type zeolite catalytic substance. If the ZSM-5-type zeolite catalytic substance supports Pt, or Pt and Pd, it is possible to increase the generated amount of liquefied petroleum gas and the content ratio of propane. For the state of the Pt and Pd supported on the ZSM-5-type zeolite catalytic substance, Pt of metal simple substance and Pd of metal simple substance may mix, Pt and Pd may alloy, or a metal simple substance of at least one among Pt and Pd and an alloy of Pt and Pd may mix.

In addition, for such a catalyst, the ZSM-5-type zeolite catalytic substance preferably contains P. If the ZSM-5-type zeolite catalytic substance contains P, since the acid strength of the ZSM-5-type zeolite catalytic substance can be properly controlled, it is possible to increase the generated amount of liquefied petroleum gas and the content ratio of propane.

In addition, for such a catalyst, the ratio (mole number of SiO₂/ mole number of Al₂O₃) of the mole number of SiO₂ relative to the mole number of Al₂O₃ contained in the ZSM-5-type zeolite catalytic substance is preferably 20 or more and 60 or less. By replacing a part of the silicon atoms in the silicates constituting the zeolite skeleton of the ZSM-5-type zeolite catalytic substance with aluminum atoms, since the aluminum atoms serve as acid sites, the zeolite catalytic substance exhibits a function as a solid acid. If the above ratio is 60 or less, since the acid sites of the ZSM-5-type zeolite catalytic substance increase, it is possible to increase the generated amount of liquefied petroleum gas and the content ratio of propane. In addition, if the above ratio is 20 or more, it is possible to suppress catalyst degradation such as coking caused by the acid sites becoming too abundant.

The heating temperature of the LPG generation catalyst is appropriately set according to the type of LPG generation catalyst, the supplied amount of the mixed gas, etc. For example, the heating temperature of the LPG generation catalyst is 240°C or higher and 350°C or lower.

For the pressure of the liquefied petroleum gas synthesis reaction, the lower limit value is preferably 2.0 MPa or more, more preferably 3.0 MPa or more, and even more preferably 3.5 MPa or more, and the upper limit value is preferably 6.0 MPa or less, more preferably 5.5 MPa or less, and even more preferably 5.0 MPa or less.

At this time, at least the liquefied petroleum gas which is the useful hydrocarbon is contained in the first gas generated in the useful hydrocarbon generating step S10. In addition to the liquefied petroleum gas, the first gas contains hydrocarbons, carbon dioxide, carbon monoxide and hydrogen that are unreacted substances of the useful hydrocarbon generating step S10 and the dry reforming step S30 and water, methanol, dimethyl ether, C1 to C2 hydrocarbons, C5 or higher hydrocarbons, etc., which are byproducts of these steps.

The content ratio of the liquefied petroleum gas contained in the first gas can be appropriately adjusted according to the conditions of the liquefied petroleum gas synthesis reaction such as the heating temperature of the LPG generation catalyst and the supplied amount of the mixed gas.

In the case of the useful hydrocarbon being liquefied petroleum gas, hydrocarbons other than C3 to C4 hydrocarbons are preferably contained in the dry reforming recycle gas, C1 to C2 hydrocarbons are more preferably contained therein, and ethane is even more preferably contained therein. If the dry reforming recycle gas contains the above-mentioned substances, the yield of liquefied petroleum gas drastically improves.

For example, in the case of producing liquid hydrocarbons as the useful hydrocarbons, the liquid hydrocarbon synthesis reaction is performed using the useful hydrocarbon generation catalyst (liquid hydrocarbon generation catalyst) for synthesizing the liquid hydrocarbons from the carbon monoxide and hydrogen contained in the mixed gas in the useful hydrocarbon generating step S10. In the useful hydrocarbon generating step S10, by heating the liquid hydrocarbon generation catalyst while supplying the mixed gas to the liquid hydrocarbon generation catalyst, the carbon monoxide and hydrogen in the supplied mixed gas react on the liquid hydrocarbon generation catalyst to generate the first gas containing the liquid hydrocarbons.

From the viewpoint of increasing the generated amount of liquid hydrocarbons, the liquid hydrocarbon generation catalyst is preferably an FT (Fischer Tropsch) synthesis catalyst.

As the FT synthesis catalyst, it is preferable to support at least one type of metal having activity against the FT reaction on the support. The support is preferably Al₂O₃ or SiO₂. In addition, the metal having activity against the FT reaction is preferably Ru, Co, Fe or Ni, and is more preferably Ru or Co. In the case of the above metal being Ru, the FT synthesis catalyst preferably contains Ru in 0.5% by mass or more and 5.0% by mass or less in terms of the metal. In the case of the above metal being Co, the FT synthesis catalyst preferably contains Co in 5.0% by mass or more and 40.0% by mass or less in terms of the metal.

In addition, the FT synthesis catalyst is a catalyst structure exemplified as an ideal example of the above-mentioned dry reforming catalyst, and may be a structure in which Co is included inside.

The heating temperature of the liquid hydrocarbon generation catalyst is appropriately set according to the type of liquid hydrocarbon generation catalyst, the supplied amount of the mixed gas, etc. For example, the heating temperature of the liquid hydrocarbon generation catalyst is 200°C or higher and 350°C or lower, preferably 210°C or higher and 310°C or lower, and more preferably 220°C or higher and 290°C or lower.

The pressure of the liquid hydrocarbon synthesis reaction is preferably 0.5 MPa or more and 10.0 MPa or less, more preferably 0.7 MPa or more and 7.0 MPa or less, and even more preferably 0.8 MPa or more and 5.0 MPa or less.

At this time, at least the liquid hydrocarbons which are the useful hydrocarbon are contained in the first gas generated in the useful hydrocarbon generating step S10. In addition to the liquid hydrocarbons, the first gas contains hydrocarbons, carbon dioxide, carbon monoxide and hydrogen that are unreacted substances of the useful hydrocarbon generating step S10 and the dry reforming step S30, and water, C4 or lower hydrocarbons, etc., which are byproducts of these steps.

The content ratio of liquid hydrocarbons contained in the first gas can be appropriately adjusted according to the conditions of the liquid hydrocarbon synthesis reaction such as the heating temperature of the liquid hydrocarbon generation catalyst and the supplied amount of the mixed gas.

In the case of the useful hydrocarbon being liquid hydrocarbons, C4 or lower hydrocarbons are preferably contained in the dry reforming recycle gas, and C4 or lower hydrocarbons and carbon monoxide are more preferably contained therein. If the dry reforming recycle gas contains the above-mentioned substances, the yield of the liquid hydrocarbon drastically improves.

### (Useful Hydrocarbon Production Device)

Next, a useful hydrocarbon production device according to an embodiment will be described. The useful hydrocarbon production device according to the embodiment is a device which performs the useful hydrocarbon production method of the above embodiment.

The useful hydrocarbon production device includes a useful hydrocarbon generating unit, a recycling unit, and a dry reforming unit.

The useful hydrocarbon generating unit constituting the useful hydrocarbon production device mainly performs the above-mentioned useful hydrocarbon generating step S10. A mixed gas containing carbon monoxide and hydrogen is supplied to the useful hydrocarbon generating unit. The useful hydrocarbon generating unit includes a useful hydrocarbon generation catalyst (not shown) and a heater (not shown) that heats the useful hydrocarbon generation catalyst. For example, the heater is a heating furnace. The useful hydrocarbon generation catalyst is heated to a predetermined temperature by the heater.

If the mixed gas is supplied to the useful hydrocarbon generating unit including the useful hydrocarbon generation catalyst in a heated state, the useful hydrocarbon generating unit causes the carbon monoxide and hydrogen in the mixed gas to react on the useful hydrocarbon generation catalyst to generate the first gas containing the target useful hydrocarbon. In this way, the useful hydrocarbon synthesis reaction is carried out on the mixed gas in the useful hydrocarbon generating unit.

The recycling unit constituting the useful hydrocarbon production device mainly performs the above-mentioned recycling step S20. The recycling unit separates the dry reforming recycle gas from the first gas generated in the useful hydrocarbon generating unit, and supplies the dry reforming recycle gas to the dry reforming unit described later.

The dry reforming unit constituting the useful hydrocarbon production device performs mainly the above-mentioned dry reforming step S30. The dry reforming recycle gas is supplied from the recycling unit to the dry reforming unit. The dry reforming unit includes a dry reforming catalyst (not shown), and a heater (not shown) for heating the dry reforming catalyst. For example, the heater is a heating furnace. The dry reforming catalyst is heated to a predetermined temperature by the heater.

If the dry reforming recycle gas is supplied to the dry reforming unit including the dry reforming catalyst in the heated state, the dry reforming unit causes the lower hydrocarbon-containing gas and carbon dioxide in the dry reforming recycle gas to react on the dry reforming catalyst to generate the second gas containing carbon monoxide and hydrogen. In this way, the dry reforming is performed on the dry reforming recycle gas in the dry reforming unit.

The second gas containing carbon monoxide and hydrogen is supplied to the useful hydrocarbon generating unit. The carbon monoxide and hydrogen supplied to the useful hydrocarbon generating unit can be used as raw materials of the useful hydrocarbon synthesis reaction carried out in the useful hydrocarbon generating unit.

As described above, the recycling unit supplies, to the dry reforming unit, the dry reforming recycle gas containing carbon dioxide and lower hydrocarbon-containing gas which are impurities (off gas) in the first gas generated in the useful hydrocarbon generating unit, and can reuse the carbon monoxide and hydrogen generated in the dry reforming unit in the useful hydrocarbon generating unit. In this way, the useful hydrocarbon production device of the embodiment can stably suppress the discharge amount of carbon dioxide over a long period of time, due to conducting recycle accompanying chemical conversion within the system of the carbon dioxide generated as a byproduct. In addition, since the recycling unit adjusts the ratio of the carbon dioxide and lower hydrocarbon-containing gas which are byproducts separated from the first gas generated in the useful hydrocarbon generating unit, and supplies the carbon dioxide and lower hydrocarbon-containing gas as raw materials of the dry reforming unit, degradation of the catalyst due to the coking is suppressed, and it is possible to stably improve the yield of the useful hydrocarbon over a long period of time.

The lower hydrocarbon-containing gas in the dry reforming recycle gas supplied to the dry reforming unit may contain hydrocarbons other than lower hydrocarbons, in addition to the lower hydrocarbons. The hydrocarbons other than lower hydrocarbons contained in the lower hydrocarbon-containing gas are not particularly limited, and are pure substances of C5 or higher hydrocarbons excluding the lower hydrocarbons and target useful hydrocarbon or a mixture of these, for example, and are preferably C5 to C10 hydrocarbons excluding the lower hydrocarbons and the target useful hydrocarbon. At this time, the ratio of the mole number of the lower hydrocarbons relative to the mole number of C5 or higher hydrocarbons excluding the lower hydrocarbons and target useful hydrocarbon in the lower hydrocarbon-containing gas (lower hydrocarbons/C5 or higher hydrocarbons excluding lower hydrocarbons and useful hydrocarbon) is preferably 1.0 or higher. If the dry reforming recycle gas containing the lower hydrocarbon-containing gas of such a composition is supplied to the dry reforming unit, the coking of the dry reforming catalyst is effectively suppressed, and thus the yield of the useful hydrocarbon generated in the useful hydrocarbon generating unit improves.

In addition, the recycling unit preferably adjusts the ratio (M_{HC}/M_{CO2}) of the carbon mole number M_{HC} of the lower hydrocarbon-containing gas relative to the carbon mole number M_{CO2} of carbon dioxide in the dry reforming recycle gas to be supplied to the dry reforming unit.

When the carbon mole ratio (M_{HC/}M_{CO2}) in the dry reforming unit is large, there is a possibility of causing the coking occurrence and the carbon dioxide deficiency in the dry reforming unit. For this reason, when the carbon mole ratio (M_{HC/}M_{CO2}) in the dry reforming unit is large, if the recycling unit decreases the ratio (M_{HC/}M_{CO2}) in the dry reforming recycle gas to be supplied to the dry reforming unit, it is possible to suppress the coking occurrence and the carbon dioxide deficiency in the dry reforming unit.

In this way, by the recycling unit adjusting the ratio (M_{HC/}M_{CO2}) in the dry reforming recycle gas to be supplied to the dry reforming unit according to the carbon mole ratio (M_{HC}/M_{CO2}) in the dry reforming unit, the target useful hydrocarbon can be more efficiently produced. If adjusting the ratio (M_{HC/}M_{CO2}) in the dry reforming unit preferably to 1.30 or less, and more preferably 1.10 or less, the above-mentioned effect can be further improved. In addition, if adjusting the ratio (M_{HC/}M_{CO2}) in the dry reforming unit preferably to 0.25 or more, and more preferably 0.40 or more, it is possible to suppress a lower hydrocarbon-containing gas deficiency, and thus improve the efficiency of dry reforming, and possible to further suppress the discharge amount of carbon dioxide.

In addition, the CO₂ concentration of the dry reforming recycle gas supplied to the dry reforming unit by the recycling unit is preferably 40% or more, more preferably 50% or more, and even more preferably 60% or more. When the CO₂ concentration of the dry reforming recycle gas is 40% or more, it is possible to sufficiently suppress the coking occurrence and the carbon dioxide deficiency in the dry reforming unit. As a result thereof, since the existence ratio of carbon monoxide to hydrogen in the second gas increases, the generation efficiency of the useful hydrocarbon improves.

In addition, the useful hydrocarbon production device of the embodiment preferably further includes a raw material supplying unit that supplies at least one of hydrogen and carbon monoxide that is obtained by the reverse shift reaction to the useful hydrocarbon generating unit. The raw material supplying unit mainly performs the above-mentioned raw material supplying step S40. The hydrogen and carbon monoxide supplied from the raw material supplying unit to the useful hydrocarbon generating unit can be used as raw materials of the useful hydrocarbon synthesis reaction performed in the useful hydrocarbon generating unit.

The carbon monoxide is generated from carbon dioxide and hydrogen by way of the reverse shift reaction. In addition, hydrogen is generated from electrolysis of water, etc., for example. In this way, the raw material supplying unit can stably supply hydrogen and carbon monoxide to the useful hydrocarbon generating unit.

In addition, the useful hydrocarbon production device of the embodiment preferably further includes a hydrogen recycling unit that separates hydrogen from the first gas generated by the useful hydrocarbon generating unit, and supplies the hydrogen to the useful hydrocarbon generating unit. The hydrogen recycling unit performs mainly the above-mentioned hydrogen recycling step S50. If supplying hydrogen which is an impurity in the first gas discharged from the useful hydrocarbon generating unit to the useful hydrocarbon generating unit by the hydrogen recycling unit, it is possible to suppress the hydrogen deficiency in the useful hydrocarbon generating unit. For this reason, the target useful hydrocarbon can be efficiently produced over a long period of time. In addition, due to reusing the hydrogen discharged from the useful hydrocarbon generating unit as a raw material of the useful hydrocarbon generating unit, the environmental burden can be reduced.

In addition, the hydrogen recycling unit preferably adjusts the supplied amount of hydrogen to be supplied to the useful hydrocarbon generating unit according to the carbon number of the useful hydrocarbon generated in the useful hydrocarbon generating unit, and more preferably adjusts the supplied amount of hydrogen to be supplied to the useful hydrocarbon generating unit, according to the carbon number of the useful hydrocarbon generated in the useful hydrocarbon generating step S10 and the mole number M_{H} of hydrogen in the useful hydrocarbon generating step S10.

For example, in the case of producing liquefied petroleum gas as the useful hydrocarbon, if the mole number M_{H} of hydrogen in the useful hydrocarbon generating unit is small, there is a possibility of causing the hydrogen deficiency in the useful hydrocarbon generating unit. For this reason, when the mole number M_{H} of hydrogen in the useful hydrocarbon generating unit is small, if the hydrogen recycling unit increases the supplied amount of hydrogen to be supplied to the useful hydrocarbon generating unit, it is possible to suppress the hydrogen deficiency in the useful hydrocarbon generating unit.

In this way, it is possible to more efficiently produce the target useful hydrocarbon by adjusting, in the hydrogen recycling unit, the supplied amount of hydrogen to be supplied to the useful hydrocarbon generating unit according to the mole number M_{H} of hydrogen in the useful hydrocarbon generating unit, such as the hydrogen recycling unit increasing the supplied amount of hydrogen to be supplied to the useful hydrocarbon generating unit when the mole number M_{H} of hydrogen in the useful hydrocarbon generating unit is small.

In addition, the hydrogen recycling unit preferably adjusts the supplied amount of hydrogen to be supplied to the useful hydrocarbon generating unit according to the mole ratio (M_{H}/M_{CO}) of the mole number M_{H} of hydrogen relative to the mole number M_{CO} of carbon monoxide in the useful hydrocarbon generating unit.

Regarding the mole ratio (M_{H}/M_{CO}) of the useful hydrocarbon generating unit, if the proportion of hydrogen relative to carbon monoxide is small, there is a possibility of leading to the hydrogen deficiency. For this reason, when the mole ratio (M_{H}/M_{CO}) in the useful hydrocarbon generating unit is small, if the hydrogen recycling unit increases the supplied amount of hydrogen to be supplied to the useful hydrocarbon generating unit, it is possible to suppress the hydrogen deficiency in the useful hydrocarbon generating unit. In this way, by the hydrogen recycling unit adjusting the supplied amount of hydrogen to be supplied to the useful hydrocarbon generating unit according to the mole ratio (M_{H}/M_{CO}) in the useful hydrocarbon generating unit, it is possible to more reliably suppress the hydrogen deficiency in the useful hydrocarbon generating unit.

In addition, the useful hydrocarbon production device of the embodiment may further include an adjusting unit that adjusts the mole ratio (M_{H}/M_{CO}) of the mole number M_{H} of hydrogen relative to the mole number M_{CO} of carbon monoxide in the second gas obtained in the dry reforming unit. The adjusting unit performs mainly the above-mentioned adjusting step.

The adjusting unit adjusts the mole ratio (M_{H}/M_{CO}) in the second gas discharged from the dry reforming unit. The adjusting unit is preferably a steam drum. The steam drum supplies steam to the dry reforming unit to adjust the mole ratio (M_{H}/M_{CO}) in the second gas to 1 or more and 3 or less. In the adjusting unit, the mole ratio (M_{H}/M_{CO}) in the second gas is adjusted according to the conditions of the useful hydrocarbon synthesis reaction conducted in the useful hydrocarbon generating unit.

If the second gas having the mole ratio (M_{H}/M_{CO}) adjusted by the adjusting unit is supplied to the useful hydrocarbon generating unit, it is possible to suppress the hydrogen deficiency due to the useful hydrocarbon generating unit. For this reason, the target useful hydrocarbon can be efficiently produced over a long period of time.

In addition, the useful hydrocarbon production device of the embodiment may further include a combusting unit that separates a substance containing carbon atoms from the first gas, and combusts the substance containing carbon atoms to generate carbon dioxide. The combusting unit performs mainly the above-mentioned combusting step. In this case, the recycling unit supplies the dry reforming recycle gas and the carbon dioxide generated in the combusting unit to the dry reforming unit.

The combusting unit separates the substance containing carbon atoms in the first gas discharged from the useful hydrocarbon generating unit from the first gas, and combusts the separated substance containing carbon atoms to generate carbon dioxide. It should be noted that carbon dioxide may be included or may not be included in the substance containing carbon atoms to be combusted in the combusting unit.

In addition to the dry reforming recycle gas, the recycling unit also supplies the carbon dioxide generated in the combusting unit to the dry reforming unit. The amount of carbon dioxide supplied to the dry reforming unit can be increased by the combusting unit. For this reason, since the coking occurrence and the carbon dioxide deficiency in the dry reforming unit can be further suppressed, the target useful hydrocarbon can be efficiently produced over a long period of time. In addition, it is possible to recover the heat generated in the combusting unit, and utilize the heat in the useful hydrocarbon generating unit and/or the dry reforming unit.

In addition, it is preferable to adjust the combustion rate of the combusting unit according to the proportion of hydrocarbons to carbon dioxide supplied to the dry reforming unit. By changing the combustion rate of the combusting unit, it is possible to adjust the amount of carbon dioxide generated in the combusting unit. For this reason, since it is possible to adjust the combustion rate of the combusting unit to adjust the generated amount of carbon dioxide according to the proportion of hydrocarbons to carbon dioxide supplied to the dry reforming unit, and possible to adjust the amount of carbon dioxide supplied to the dry reforming unit by the recycling unit, the generated amount of the target useful hydrocarbon can be increased.

If the combustion rate of the combusting unit is 3% or more, the carbon dioxide deficiency in the dry reforming unit can be sufficiently suppressed. In addition, if the combustion rate of the combusting unit is 25% or less, the proportion of hydrocarbons to carbon dioxide supplied to the dry reforming unit will be favorable. On the other hand, if the combustion rate of the combusting unit exceeds 25%, since carbon dioxide becomes excessive relative to hydrocarbons, the unreacted carbon dioxide amount becomes great, and as a result, the generated amount of the useful hydrocarbon declines. For this reason, if the combustion rate of the combusting unit is 3% or more and 25% or less, the target useful hydrocarbon can be efficiently produced over a long period of time.

In addition, trace amounts of sulfur and halogens may be mixed as impurities in the useful hydrocarbon production device of the embodiment. Since these become poisonous substances for the useful hydrocarbon generation catalyst used in the useful hydrocarbon generating unit and the dry reforming catalyst used in the dry reforming unit, there is a possibility of hindering the catalytic performance of the above catalysts, and may become the cause of damage to equipment. Therefore, the useful hydrocarbon production device of the embodiment may include a desulfurization unit, or dehalogenation unit or the like as appropriate.

In addition, the useful hydrocarbon production device of the embodiment may include a dehydrating unit that dehydrates the second gas generated by the dry reforming unit. The dehydrating unit performs mainly the above-mentioned dehydrating step. The dehydrating unit is provided between the dry reforming unit and the useful hydrocarbon generating unit. By the dehydrating unit dehydrating the second gas, it is possible to improve the efficiency of the useful hydrocarbon synthesis reaction performed by the useful hydrocarbon generating unit, and possible to further suppress a decline in the catalytic performance of the useful hydrocarbon generation catalyst due to moisture.

In addition, the useful hydrocarbon production device of the embodiment preferably includes a separating unit after the raw material supplying unit and before the useful hydrocarbon generating unit. The separating unit mainly performs the above-mentioned separating step. Hydrogen and carbon dioxide, water, etc. are contained as unreacted substances and byproducts in the gas generated from the raw material supplying unit. For this reason, a part or all of the hydrogen and/or carbon dioxide may be separated from carbon monoxide and recycled as appropriate to the raw material supplying unit. In addition, it is preferable to separate water from the carbon monoxide, due to being able to improve the efficiency of the useful hydrocarbon synthesis reaction, and possible to further suppress a decline in the catalytic performance of the useful hydrocarbon generation catalyst due to moisture.

In addition, the useful hydrocarbon production device of the embodiment may include a compressing unit that compresses the second gas generated by the dry reforming unit, as well as a gas containing at least one of hydrogen and carbon monoxide supplied from raw material supplying unit. The compressing unit performs mainly the above-mentioned compressing step. The compressing unit is provided after the dry reforming unit and raw material supplying unit, and before and the useful hydrocarbon generating unit. By the compressing unit compressing the second gas, etc., since the compressed second gas, et **c.** is supplied to the useful hydrocarbon generating unit, it is possible to improve the efficiency of the synthesis reaction of the target useful hydrocarbon performed by the useful hydrocarbon generating unit.

In addition, the useful hydrocarbon produced by the useful hydrocarbon production device is similar to the useful hydrocarbon produced by the above-mentioned useful hydrocarbon production method, and is preferably at least one type of hydrocarbon selected from the group consisting of olefins, aromatic hydrocarbons, gasoline, liquefied petroleum gas and liquid hydrocarbons, and is more preferably liquefied petroleum gas.

The useful hydrocarbon production device can synthesize a hydrocarbon mixture with various target useful hydrocarbons as the main component, from carbon monoxide and hydrogen, by various useful hydrocarbon synthesis reactions, with the appropriate catalysts (useful hydrocarbon generation catalyst) and reaction conditions in the useful hydrocarbon generating unit, according to the type of useful hydrocarbon to be produced. The various useful hydrocarbon synthesis reactions performed in the useful hydrocarbon generating unit have similar conditions as the useful hydrocarbon generating step S10 of the above-described useful hydrocarbon production method.

According to the above described embodiment, by using hydrogen as the main raw material of the useful hydrocarbon synthesis reaction, and further recycling carbon dioxide which is one of the principal byproducts within the system, it is possible to improve the yield of the useful hydrocarbon stably over a long period of time, and suppres the discharge amount of carbon dioxide.

Although embodiments have been explained above, the present invention is not to be limited to the above embodiments, and encompasses every embodiment included in the gist of the present disclosure and scope of the claims, and various modifications thereto are possible within the scope of the present disclosure.

### EXAMPLES

Next, examples and comparative examples will be described; however, the present invention is not to be limited to these examples.

### (Examples 1-1 to 1-6)

Liquefied petroleum gas, which is a useful hydrocarbon, was produced by the useful hydrocarbon production method including the useful hydrocarbon generating step, the recycling step, and the dry reforming step. More specifically, it was produced as follows.

In the useful hydrocarbon generating step, a fixed bed flow reactor was used. In addition, a stainless-steel reaction tube was used. The mixed gas (carbon monoxide : hydrogen = 1:2 by mole ratio) was supplied to the useful hydrocarbon generating step, and the flowrate of the mixed gas to be supplied to the useful hydrocarbon generating step was set to 37.2 ml/min. The useful hydrocarbon generation catalyst (LPG generation catalyst) was filled so as to be GHSV = 2000 (1/h), and the LPG generation catalyst was fixed by packing quartz wool above and below this. A catalyst prepared by physically mixing, in a 1:1 weight ratio, a methanol synthesis catalytic substance, and ZSM-5-type zeolite catalytic substance on which Pt and Pd are supported was used as the LPG generation catalyst, and a reduction treatment was conducted as a pretreatment on the LPG generation catalyst for 2 hours at 380°C under a hydrogen flow. The heating temperature of the LPG generation catalyst was set to 320°C. The pressure in the useful hydrocarbon generating step was set to 5.0 MPa. The first gas containing liquefied petroleum gas was generated in this way.

In the recycling step, the dry reforming recycle gas having the composition, CO₂ concentration, ratio (M_{HC}/M_{CO2}), and ratio (lower hydrocarbons / C5 or higher hydrocarbons excluding lower hydrocarbons) of the mole number of lower hydrocarbons relative to the mole number of C5 or higher hydrocarbons excluding lower hydrocarbons, shown in Table 1 was separated from the first gas and supplied to the dry reforming step.

In addition, the dry reforming step used a fixed bed flow reactor. In addition, a quartz glass reaction tube was used. The flowrate of dry reforming recycle gas to be supplied to the dry reforming step was set to 10 ml/min. By filling a dry reforming catalyst so as to make GHSV = 2170 (1/h), and packing quartz wool above and below this, the dry reforming catalyst was fixed. As the dry reforming catalyst, the above-mentioned dry reforming catalyst containing 1% by mass of Ni was used, and reduction treatment was conducted as a pretreatment on the dry reforming catalyst for 1.5 hours at 700°C under a hydrogen flow. The dry reforming step was conducted at the reaction temperature of dry reforming shown in Table 1.

The useful hydrocarbon production method was started, and immediately after starting (initial stage) and after 7 days, the second gas generated in the dry reforming step was recovered by a gas pack and analyzed by GC-TCD, and the synthesis gas (hydrogen and carbon monoxide) generated in the dry reforming step and the generated amount of carbon dioxide were measured. When the synthesis gas thereby generated immediately after starting (initial stage) and the synthesis gas generated after 7 days were respectively supplied to the useful hydrocarbon generating step, the generated amounts of liquefied petroleum gas and carbon dioxide contained in the first gas were measured. In addition, the activity decline rate of the dry reforming catalyst was calculated by the below Formula, from the change in the generated amount of carbon dioxide in the dry reforming step immediately after start (initial stage) and after 7 days. The activity decline rate represents the percentage of the carbon dioxide conversion rate which has deteriorated from immediately after start to after 7 days, relative to the initial carbon dioxide conversion rate. The results of the activity decline rate are shown in Table 1. Activity decline rate (%) = (initial carbon dioxide conversion rate (%) - post-7-day carbon dioxide conversion rate) × 100/initial carbon dioxide conversion rate (%)

Furthermore, the amount of carbon dioxide contained in the useful hydrocarbon, and the total of the discharge amount of carbon dioxide discharged to outside the system of the process after the dry reforming step were calculated.

### (Comparative Example 1-1)

Liquefied petroleum gas was produced similarly to the above-mentioned example, except for including the useful hydrocarbon generating step, but not including the recycling step and the dry reforming step. Furthermore, the carbon dioxide contained in the useful hydrocarbon, i.e. the discharge amount of carbon dioxide discharged to outside the system of the process, was measured.

The ranking of the LPG generated amount in Table 2 was defined as "excellent" for the LPG generated amount at the initial stage and after 7 days exceeding 1.72 (10⁻¹ mol/mol - raw material CO), defined as "great" for greater than 1.30 (10⁻¹ mol/mol - raw material CO) and 1.72 (10⁻¹ mol/mol - raw material CO) or less, defined as "good" for greater than 1.10 (10⁻¹ mol/mol - raw material CO) and 1.30 (10⁻¹ mol/mol - raw material CO) or less, and defined as "poor" for 1.10 (10⁻¹ mol/mol - raw material CO) or less.

In addition, the ranking of the carbon dioxide discharge amount in Table 2 was defined as "great" for the carbon dioxide discharge amount discharged to outside the process system at the initial stage and after 7 days of 2.50 (10⁻¹ mol/mol - raw material CO) or less, defined as "good" for greater than 2.50 (10⁻¹ mol/mol - raw material CO) and 3.45 (10⁻¹ mol/mol - raw material CO) or less, and defined as "poor" for greater than 3.45 (10⁻¹ mol/mol - raw material CO).

In addition, regarding the comprehensive evaluation based on these rankings, among the four items of the LPG generated amount and the carbon dioxide discharge amount at the initial stage and after 7 days, the case of one or more being the rank of excellent and the others being great was given "☆" (star), the case of all four being the rank of great was given "⊙" (bullseye), the case of 1 to 3 being the rank of great and the other(s) being good was given "o" (circle), the case of four being the rank of good was given "Δ" (triangle), and the case of one or more being the rank of poor was given "x" (cross). These results are shown in Table 2.

**[Table 1]**

| | Dry reforming recycle gas | | | | | | | | Dry reforming step | |
|---|---|---|---|---|---|---|---|---|---|---|
| | Composition (mole ratio) | | | | | CO₂ concentration (%) | Ratio (M_{HC}/M_{CO2}) (carbon mole ratio) | Lower hydrocarbons/(C5+ hydrocarbons + lower hydrocarbons) (carbon mole ratio) | Reaction temperature (°C) | Activity decline rate (%) |
| | CO₂ | CH₄ | C₂H₆ | CO | C5+ hydrocarbons | | | | | |
| Example 1-1 | 14.3 | 1.0 | 2.9 | 2.7 | 0.0 | 69 | 0.47 | 1.00 | 800 | 0 |
| Example 1-2 | 6.5 | 1.0 | 2.6 | 0.5 | 0.0 | 61 | 0.96 | 1.00 | 800 | 2 |
| Example 1-3 | 6.1 | 1.0 | 2.9 | 0.0 | 0.0 | 61 | 1.10 | 1.00 | 800 | 11 |
| Example 1-4 | 1.6 | 0.0 | 1.0 | 0.5 | 0.0 | 51 | 1.25 | 1.00 | 800 | 16 |
| Example 1-5 | 25.1 | 2.0 | 5.6 | 8.1 | 1.0 | 60 | 0.74 | 0.71 | 900 | 0 |
| Example 1-6 | 14.6 | 2.0 | 2.0 | 8.1 | 1.0 | 53 | 0.77 | 0.52 | 900 | 0 |
| Example 2-1 | 14.7 | 1.0 | 2.9 | 2.7 | 0.0 | 69 | 0.46 | 1.00 | 800 | 0 |
| Example 2-2 | 16.7 | 1.0 | 2.9 | 2.6 | 0.0 | 72 | 0.40 | 1.00 | 800 | 0 |
| Comparative Example 1-1 | No recycling | | | | | | | | | |

**[Table 2]**

| | LPG generated amount | | CO₂ discharge amount | | Combustion rate (%) | Comprehensive evaluation |
|---|---|---|---|---|---|---|
| | Initial stage | After 7 days | Initial stage | After 7 days | | |
| Example 1-1 | Great | Great | Great | Great | 0 | ⊚ |
| Example 1-2 | Great | Great | Good | Good | 0 | ○ |
| Example 1-3 | Great | Good | Good | Good | 0 | ○ |
| Example 1-4 | Good | Good | Good | Good | 0 | Δ |
| Example 1-5 | Excellent | Excellent | Great | Great | 0 | ☆ |
| Example 1-6 | Great | Great | Good | Good | 0 | ○ |
| Example 2-1 | Great | Great | Great | Great | 5 | ⊚ |
| Example 2-2 | Excellent | Excellent | Great | Great | 25 | ☆ |
| Comparative Example 1-1 | Poor | Poor | Poor | Poor | 0 | × |

### (Examples 2-1 to 2-2)

Liquefied petroleum gas was produced similarly to Examples 1-1 to 1-6 except for providing the combusting step after the useful hydrocarbon generating step and before the recycling step, and combusting the hydrocarbons other than the useful hydrocarbon in the first gas at the combustion rate shown in Table 2 to convert into carbon dioxide and supplying carbon dioxide to the recycling step, and the composition of the dry reforming recycle gas, CO₂ concentration, ratio (M_{HC/}M_{CO2}) and ratio (lower hydrocarbons/C5 or higher hydrocarbons excluding lower hydrocarbons) in accordance with Table 1. The activity decline rate, the generated amount of liquefied petroleum gas, the carbon dioxide discharge amount and the comprehensive evaluation are shown in Tables 1 and 2.

### (Examples 3-1 to 3-6)

Liquefied petroleum gas which is the useful hydrocarbon was produced by a useful hydrocarbon production method further including a raw material supplying step, in addition to the useful hydrocarbon generating step, recycling step and dry reforming step. More specifically, it was as follows.

In the raw material supply step, a reverse shift reaction catalyst was prepared and used in the production method described in PCT International Publication No. WO2018/221690. Cu was selected as the metal, and the content thereof was set to 1% by mass. A fixed bed flow reactor was used in the reverse shift reaction, and a quartz glass reaction tube was used. The hydrogen gas was supplied at 3.0 ml/min, and carbon dioxide was supplied at 1.5 ml/min. By filling the reverse shift reaction catalyst so as to make GHSV = 2140 (1/h), and packing quartz wool above and below this, the reverse shift reaction catalyst was fixed. The reaction temperature of the reverse shift reaction was set to 750°C. The generated gas was recovered by a gas pack and analyzed by GC-TCD, and the generated amount of carbon monoxide generated by the reverse shift reaction was measured.

The useful hydrocarbon generating step was conducted similarly to Examples 1-1 to 1-6, except for adding to the carbon monoxide (38% CO₂ conversion rate) obtained by the reverse shift reaction in the raw material supplying step, twice the amount (mole ratio) of hydrogen, and supplying to the useful hydrocarbon generating step.

In the recycling step, the dry reforming recycle gas having the composition, the CO₂ concentration, the ratio (M_{HC/}M_{CO2}) and the ratio (lower hydrocarbons/C5 or higher hydrocarbons excluding lower hydrocarbons) of the mole number of lower hydrocarbons relative to mole number of C5 or higher hydrocarbons excluding the lower hydrocarbons, shown in Table 3 was, separated from the first gas, and supplied to the dry reforming step.

In addition, the dry reforming step was conducted similarly to Examples 1-1 to 1-6.

The activity decline rate in the dry reforming step, the generated amount of the liquefied petroleum gas, and the carbon dioxide discharge amount were calculated similarly to Examples 1-1 to 1-6.

### (Comparative Example 3-1)

Liquefied petroleum gas was produced similarly to the above-mentioned Example 3-1, except for including the useful hydrocarbon generating step and the raw material supplying step, but not including the recycling step and the dry reforming step. The generated amount of the liquefied petroleum gas and the carbon dioxide discharge amount were calculated similarly to Comparative Example 1-1.

### (Examples 4-1 to 4-2)

Liquefied petroleum gas was produced similarly to Examples 3-1 to 3-6, except for providing the combusting step after the useful hydrocarbon generating step and before the recycling step, and combusting the hydrocarbons other than the useful hydrocarbon in the first gas at the combustion rate shown in Table 4 to convert into carbon dioxide and supplying to the recycling step, and changing the composition of the dry reforming recycle gas, the CO₂ concentration, the ratio (M_{HC/}M_{CO2}) and the ratio (lower hydrocarbons/C5 or higher hydrocarbons excluding lower hydrocarbons) in accordance with Table 3. The calculated activity decline rate, the carbon dioxide discharge amount and the generated amount of the liquefied petroleum gas are shown in Tables 3 and 4.

The ranking of the LPG generated amount in Table 4 was defined as "excellent" for the LPG generated amount at the initial stage and after 7 days exceeding 8.0 (10⁻² mol/mol - raw material CO₂), defined as "great" for greater than 6.0 (10⁻² mol/mol - raw material CO₂) and 8.0 (10⁻² mol/mol - raw material CO₂) or less, defined as "good" for greater than 5.0 (10⁻² mol/mol - raw material CO₂) and 6.0 (10⁻² mol/mol - raw material CO₂) or less, and defined as "poor" for 5.0 (10⁻² mol/mol - raw material CO₂) or less.

In addition, the ranking of the carbon dioxide discharge amount in Table 4 was defined as "great" for the carbon dioxide discharge amount discharged to outside the process system at the initial stage and after 7 days of 1.35 (10⁻¹ mol/mol - raw material CO₂) or less, defined as "good" for greater than 1.35 (10⁻¹ mol/mol - raw material CO₂) and 1.55 (10⁻¹ mol/mol - raw material CO₂) or less, and defined as "poor" for greater than 1.55 (10⁻¹ mol/mol - raw material CO₂).

In addition, regarding the comprehensive evaluation based on these rankings, among the four items of the LPG generated amount and the carbon dioxide discharge amount at the initial stage and after 7 days, the case of one or more being the rank of excellent and the others being great was given "☆" (star), the case of all four being the rank of great was given "⊙" (bullseye), the case of 1 to 3 being the rank of great and the other(s) being good was given "o" (circle), the case of all four being the rank of good was given "Δ" (triangle), and the case of one or more being the rank of poor was given "x" (cross). These results are shown in Table 4.

**[Table 3]**

| | Dry reforming recycle gas | | | | | | | | Dry reforming step | |
|---|---|---|---|---|---|---|---|---|---|---|
| | Composition (mole ratio) | | | | | CO₂ concentration (%) | Ratio (M_{HC}/M_{CO2}) (carbon mole ratio) | Lower hydrocarbons/(C5+ hydrocarbons + lower hydrocarbons) (carbon mole ratio) | Reaction temperature (°C) | Activity decline rate (%) |
| | CO₂ | CH₄ | C₂H₆ | CO | C5+ hydrocarbons | | | | | |
| Example 3-1 | 14.3 | 1.0 | 2.9 | 0.0 | 0.0 | 79 | 0.47 | 1.00 | 800 | 0 |
| Example 3-2 | 7.3 | 1.0 | 2.9 | 0.0 | 0.0 | 65 | 0.93 | 1.00 | 800 | 2 |
| Example 3-3 | 6.4 | 1.0 | 2.9 | 0.0 | 0.0 | 63 | 1.04 | 1.00 | 800 | 13 |
| Example 3-4 | 1.6 | 0.0 | 1.0 | 0.9 | 0.0 | 45 | 1.27 | 1.00 | 800 | 14 |
| Example 3-5 | 26.2 | 1.8 | 5.3 | 7.6 | 1.0 | 64 | 0.80 | 0.69 | 900 | 0 |
| Example 3-6 | 26.2 | 1.8 | 2.5 | 7.6 | 1.0 | 69 | 0.51 | 0.56 | 900 | 0 |
| Example 4-1 | 14.7 | 1.0 | 2.9 | 4.1 | 0.0 | 65 | 0.46 | 1.00 | 800 | 0 |
| Example 4-2 | 16.7 | 1.0 | 2.9 | 4.1 | 0.0 | 68 | 0.40 | 1.00 | 800 | 0 |
| Comparative Example 3-1 | No recycling | | | | | | | | | |

**[Table 4]**

| | LPG generated amount | | CO₂ discharge amount | | Combustion rate (%) | Comprehensive evaluation |
|---|---|---|---|---|---|---|
| | Initial stage | After 7 days | Initial stage | After 7 days | | |
| Example 3-1 | Great | Great | Great | Great | 0 | ⊚ |
| Example 3-2 | Great | Great | Great | Great | 0 | ⊚ |
| Example 3-3 | Great | Good | Great | Great | 0 | ○ |
| Example 3-4 | Good | Good | Good | Good | 0 | Δ |
| Example 3-5 | Excellent | Excellent | Great | Great | 0 | ☆ |
| Example 3-6 | Excellent | Excellent | Great | Great | 0 | ☆ |
| Example 4-1 | Excellent | Excellent | Great | Great | 5 | ☆ |
| Example 4-2 | Excellent | Excellent | Great | Great | 25 | ☆ |
| Comparative Example 3-1 | Poor | Poor | Poor | Poor | 0 | × |

In Examples 5-1 to 5-4, propylene which is an olefin, aromatic hydrocarbons, gasoline and liquid hydrocarbon were produced in the useful hydrocarbon generating step, by the production method including the useful hydrocarbon generating step, the recycling step and the dry reforming step. The useful hydrocarbon generating step in each example was executed as follows, and otherwise, the recycling step and the dry reforming step were conducted similarly to Examples 1-1 to 1-6 at the conditions shown in Table 5. The carbon dioxide concentration and the ratio (M_{HC/}M_{CO2}) of the dry reforming recycle gas were set to the values shown in Table 5.

### (Example 5-1)

Propylene, which is an olefin, was produced similarly to the above Examples 1-1 to 1-6, except for using a catalyst made by physically mixing, in a 1:1 weight ratio, a methanol synthesis catalytic substance and a ZSM-5-type zeolite catalytic substance on which P is supported as the useful hydrocarbon generation catalyst (olefin generation catalyst) filled in the useful hydrocarbon generating step, and setting the reaction temperature to 320°C, GHSV=2000 h⁻¹, the pressure to 5.0 MPa, and the supplied H₂/CO ratio to 2.0.

### (Example 5-2)

Aromatic hydrocarbons (mixture of benzene, toluene, xylene and trimethylbenzene) were produced similarly to the above Examples 1-1 to 1-6, except for using a catalyst made by physically mixing, in a 1:1 weight ratio, a methanol synthesis catalytic substance and a ZSM-5-type zeolite catalytic substance (ZnCr₂O₄/ZSM-5) in which 1 wt% of ZnCr₂O₄ is physically mixed as the useful hydrocarbon generation catalyst (aromatic hydrocarbon generation catalyst) filled in the useful hydrocarbon generating step, and setting the reaction temperature to 350°C, GHSV=500 h⁻¹, the pressure to 5.0 MPa, and the supplied H_{2/}CO ratio to 2.5.

### (Example 5-3)

Gasoline was produced similarly to the above Examples 1-1 to 1-6, except for using a catalyst made by physically mixing, in a 1:1 weight ratio, a methanol synthesis catalytic substance and a ZSM-5-type zeolite catalytic substance as the useful hydrocarbon generation catalyst (gasoline generation catalyst) filled in the useful hydrocarbon generating step, and setting the reaction temperature to 370°C, GHSV=3700 h⁻¹, the pressure to 5.0 MPa, and the supplied H_{2/}CO ratio to 2.0.

### (Example 5-4)

Liquid hydrocarbon was produced similarly to the above Examples 1-1 to 1-6, except for using 0.5 wt% Co-supporting SiO₂ (FT synthesis catalyst) as the useful hydrocarbon generation catalyst (liquid hydrocarbon generation catalyst) filled in the useful hydrocarbon generating step, and setting the reaction temperature to 230°C, GHSV=3000 h⁻¹, the pressure to 2.0 MPa, and the supplied H_{2/}CO ratio to 2.0.

### (Comparative Examples 5-1 to 5-4)

Propylene which is an olefin, aromatic hydrocarbons (mixture of benzene, toluene, xylene and trimethylbenzene), gasoline and liquid hydrocarbon were respectively produced in the useful hydrocarbon generating step similarly to Examples 5-1 to 5-4, except for including the useful hydrocarbon generating step, but not including the recycling step and the dry reforming step.

The generated amounts of the target useful hydrocarbon and carbon dioxide contained in the first gas, and the generated amounts of carbon dioxide and carbon monoxide contained in the second gas were analyzed by the same method as described above, and the CO conversion ratio of the useful hydrocarbon generating step, carbon yield of target useful hydrocarbon, as well as the carbon dioxide discharge amount discharged outside the process system were calculated. The results are shown in Table 5.

**[Table 5]**

| | Useful hydrocarbon | Dry reforming recycle gas | | | Dry reforming step | | Useful hydrocarbon generating step | | |
|---|---|---|---|---|---|---|---|---|---|
| | | CO₂ concentration (%) | Ratio (M_{HC}/M_{CO2}) (carbon mole ratio) | Lower hydrocarbons/(C5+ hydrocarbons + lower hydrocarbons) (carbon mole ratio) | Reaction temperature (°C) | CO₂ conversion rate (%) | CO conversion rate (%) | Carbon yield of useful hydrocarbon (carbon mol - target substance / carbon mol - raw material CO) | CO₂ discharge amount (mol - CO₂/mol - raw material CO) |
| Example 5-1 | Propylene | 46 | 1.02 | 0.75 | 800 | 96 | 95 | 0.46 | 0.01 |
| Example 5-2 | Aromatic hydrocarbon | 40 | 0.37 | 0.85 | 800 | 50 | 60 | 0.23 | 0.16 |
| Example 5-3 | Gasoline | 46 | 1.01 | 1.00 | 800 | 95 | 93 | 0.46 | 0.02 |
| Example 5-4 | Liquid hydrocarbon | 66 | 0.51 | 1.00 | 800 | 70 | 60 | 0.52 | 0.02 |
| Comparative Example 5-1 | Propylene | No recycling | | | | | 95 | *0.27* | 0.33 |
| Comparative Example 5-2 | Aromatic hydrocarbon | | | | | | 60 | 0.14 | 0.33 |
| Comparative Example 5-3 | Gasoline | | | | | | 93 | 0.28 | 0.33 |
| Comparative Example 5-4 | Liquid hydrocarbon | | | | | | 60 | 0.48 | 0.07 |

As shown in Tables 1 to 5, the above Examples could stably improve the yield of various types of useful hydrocarbons over a long period of time, due to including the useful hydrocarbon generating step, the recycling step and the dry reforming step. Furthermore, the discharge amount of carbon dioxide could be suppressed. In addition, the yield of the useful hydrocarbon could be further improved by including the combusting step. On the other hand, since the above comparative examples do not include the recycling step and the dry reforming step, the yield of the useful hydrocarbon was poor. Furthermore, the discharge amount of carbon dioxide also could not be suppressed.

### EXPLANATION OF REFERENCE NUMERALS

- S10: useful hydrocarbon generating step
- S20: recycling step
- S30: dry reforming step
- S40: raw material supplying step
- S50: hydrogen recycling step

## Claims

1. A useful hydrocarbon production method comprising:
a useful hydrocarbon generating step of generating a first gas containing a useful hydrocarbon from a mixed gas containing carbon monoxide and hydrogen;
a recycling step of separating a dry reforming recycle gas containing at least carbon dioxide and a lower hydrocarbon-containing gas containing a C1 to C4 lower hydrocarbon from the first gas; and
a dry reforming step of generating a second gas containing carbon monoxide and hydrogen from the lower hydrocarbon-containing gas and carbon dioxide in the dry reforming recycle gas supplied from the recycling step, and supplying the second gas to the useful hydrocarbon generating step.

2. The useful hydrocarbon production method according to claim 1, further comprising a raw material supplying step of supplying at least one of hydrogen and carbon monoxide that is obtained by reverse shift reaction to the useful hydrocarbon generating step.

3. The useful hydrocarbon production method according to claim 1, wherein the recycling step adjusts a ratio (M_{HC}/M_{CO2}) of a carbon mole number M_{HC} of the lower hydrocarbon-containing gas relative to a carbon mole number M_{CO2} of carbon dioxide in the dry reforming recycle gas to be supplied to the dry reforming step.

4. The useful hydrocarbon production method according to claim 1, wherein a CO₂ concentration contained in the dry reforming recycle gas is 40% or more.

5. The useful hydrocarbon production method according to claim 1, further comprising a hydrogen recycling step of separating hydrogen from the first gas and supplying the hydrogen to the useful hydrocarbon generating step.

6. The useful hydrocarbon production method according to claim 5, wherein the hydrogen recycling step adjusts a supplied amount of hydrogen to be supplied to the useful hydrocarbon generating step, according to a molar amount of hydrogen supplied from the raw material supplying step and the dry reforming step.

7. The useful hydrocarbon production method according to claim 1, further comprising an adjusting step of adjusting a mole ratio (M_{H}/M_{CO}) of a mole number M_{H} of hydrogen relative to a mole number M_{CO} of carbon monoxide in the second gas obtained in the dry reforming step.

8. The useful hydrocarbon production method according to claim 1, further comprising a combusting step of separating a substance containing carbon atoms from the first gas, and combusting the substance containing carbon atoms to generate carbon dioxide,
wherein the recycling step supplies the dry reforming recycle gas and carbon dioxide generated in the combusting step to the dry reforming step.

9. The useful hydrocarbon production method according to any one of claims 1 to 8, wherein the useful hydrocarbon is at least one type of hydrocarbon selected from the group consisting of olefins, aromatic hydrocarbons, gasoline, liquefied petroleum gas and liquid hydrocarbons.

10. The useful hydrocarbon production method according to any one of claims 1 to 8, wherein the useful hydrocarbon is liquefied petroleum gas.

11. The useful hydrocarbon production method according to any one of claims 1 to 8, wherein the useful hydrocarbon is liquefied petroleum gas, and the dry reforming recycle gas contains a hydrocarbon other than C3 to C4 hydrocarbons.

12. The useful hydrocarbon production method according to any one of claims 1 to 8, wherein the useful hydrocarbon is an olefin, and the dry reforming recycle gas contains a hydrocarbon other than a target olefin.

13. The useful hydrocarbon production method according to claim 12, wherein the dry reforming recycle gas further contains carbon monoxide.

14. The useful hydrocarbon production method according to any one of claims 1 to 8, wherein the useful hydrocarbon is an aromatic hydrocarbon, and the dry reforming recycle gas contains a hydrocarbon other than a target aromatic hydrocarbon.

15. The useful hydrocarbon production method according to claim 14, wherein the dry reforming recycle gas further contains carbon monoxide.

16. The useful hydrocarbon production method according to any one of claims 1 to 8, wherein the useful hydrocarbon is gasoline, and the dry reforming recycle gas contains a hydrocarbon other than gasoline.

17. The useful hydrocarbon production method according to claim 16, wherein the dry reforming recycle gas further contains carbon monoxide.

18. The useful hydrocarbon production method according to any one of claims 1 to 8, wherein the useful hydrocarbon is a liquid hydrocarbon, and the dry reforming recycle gas contains C4 or lower hydrocarbons.

19. The useful hydrocarbon production method according to claim 18, wherein the dry reforming recycle gas further contains carbon monoxide.

20. A useful hydrocarbon production device, comprising:
a useful hydrocarbon generating unit that generates a first gas containing a useful hydrocarbon from a mixed gas containing carbon monoxide and hydrogen;
a recycling unit that separates a dry reforming recycle gas containing at least carbon dioxide and a lower hydrocarbon-containing gas containing a C1 to C4 lower hydrocarbon from the first gas; and
a dry reforming unit that generates a second gas containing carbon monoxide and hydrogen from the lower hydrocarbon-containing gas and carbon dioxide in the dry reforming recycle gas supplied from the recycling unit, and supplies the second gas to the useful hydrocarbon generating unit.
